# EUROPEAN PATENT APPLICATION

(11) **EP 2 649 996 A1**
(43) Date of publication of application: **16.10.2013**
(21) Application number: 12384004.3
(22) Date of filing: 11.04.2012
(51) Int. Cl.: A61K 31/4184, A61P 9/12

(54) **Crystalline forms of sartans like telmisartan with beta blockers**

(71) Applicant: Laboratorios del. Dr. Esteve, S.A., 08041 Barcelona (ES)
(72) Inventor: Plata Salaman, Carlos Ramon, 08950 Esplugues de Llobregat (Barcelona) (ES); Tesson, Nicolas, 08902 L'Hospitalet de Llobregat (Barcelona) (ES); Jiménez Gonzalez, Carmen, 08849 Sant Climent de Llobregat (Barcelona) (ES); Vaiana, LEA, 08028 Barcelona (ES)
(74) Representative: Peters, Hajo

(57) **Abstract**

The present invention relates to crystalline forms comprising telmisartan and at least one beta blocker, especially a selective β₁ receptor blocker, especially to salts or co-crystals of telmisartan and a beta blocker, preferably selected from selective β₁ receptor blockers, processes for preparation of the same and their use as medicament or in pharmaceutical formulations, more particularly for the treatment of hypertension, heart failure and myocardial infarction.

## Description

The present invention relates to crystalline forms comprising a sartan like telmisartan and at least one beta blocker, especially a selective β₁ receptor blocker, especially to salts or co-crystals of a sartan like telmisartan and a beta blocker, preferably selected from selective β₁ receptor blockers, processes for preparation of the same and their use as medicament or in pharmaceutical formulations, more particularly for the treatment of hypertension, heart failure and myocardial infarction.

Cardiovascular Diseases (CVDs) include diseases of the heart, vascular diseases of the brain and diseases of blood vessels. CVDs are responsible for over 17.3 million deaths per year and are the leading causes of death in the world (WHO, 2008; Causes of death 2008, World Health Organization, Geneva).

The underlying disease process in CVDs is known as atherosclerosis. Atherosclerosis is a complex pathological process in the walls of blood vessels that develops over many years. It consists on deposits of material inside the walls of arteries, in a way that, when grow, these deposits difficult the blood flow; also eventually, the plaque can rupture, triggering the formation of a blood clot. When this occurs, symptoms appear whose nature depends on the location of the lesion (e.g. stroke in the brain, myocardial infarction in the heart).

Most factors that promote the process of atherosclerosis are identified, and hypertension is considered a major risk factor for atherosclerosis, and therefore for CVDs.

Hypertension is a highly prevalent condition; globally 26.4% of the adult population had hypertension, being a leading cause of morbidity and mortality (Kearney et al., (2005), Lancet; 365: 217-23). The disease 'per se' is asymptomatic and its identification is usually through screening. If symptoms appear, the damaging effects of the condition have already been established. Hypertension is quantitatively the most important risk factor for premature CVD, increasing the risk for a variety of CVDs, including stroke, coronary artery disease/myocardial infarction (MI), heart failure (HF), and peripheral vascular disease, and vice versa. Thus, patients with established CVD often suffer from hypertension.

Heart failure (HF) is a complex clinical syndrome of symptoms and signs that suggest that the efficiency of the heart as a pump is being impaired. This is often caused by structural or functional abnormalities of the heart, with hypertension being one of the major causes. Many patients with heart failure had a myocardial infarction (MI) in the past.

Myocardial infarction (MI) reflects cell death of cardiac myocytes as a result of the interruption of blood supply to a part of the heart. MI is most commonly due to occlusion of a coronary artery following the rupture of an atherosclerotic plaque in the wall of an artery leading to the classical symptoms of acute MI (sudden chest pain, shortness of breath, nausea, vomiting, palpitations, sweating, and anxiety). The incidence of MI increases with age; however, the actual incidence is dependent on predisposing risk factors. Among those, hypertension is listed as primary.

Although it is occasionally possible to identify a specific cause for hypertension in some patients, blood pressure (BP) elevation is usually multifactorial, making it very difficult, if not impossible, to normalize pressure by interfering with only a single pressure mechanism. In addition, drug therapy directed at any one component routinely evokes compensatory (counter-regulatory) responses that reduce the magnitude of response, even if it was accurately directed at the predominant patho-physiological mechanism. As a consequence, only limited BP reduction is seen with all available antihypertensive agents used in monotherapy (Law et al., (2003); BMJ; 326: 1427-35). Clinical trials thus have shown that achieving BP targets is often not possible with just a single agent. The aggregation of available data suggests that at least 75% of patients will require combination therapy to achieve contemporary BP targets.

In this context, there are six major classes of agents: diuretics, calcium antagonists, angiotensin-converting-enzyme (ACE) inhibitors, angiotensin II receptor (ARA II) antagonists, direct rennin inhibitors and beta-blockers, whose mechanism of action affects one of three main pressor mechanisms listed above.

The renin-angiotensin system is important in control of BP. Its blockade is a widely accepted mean of controlling hypertension and treating heart failure. Angiotensin, which is the major effector hormone of this system, produces fluid and sodium retention and increments in vascular tone (vasoconstriction). All these are pressor mechanisms, so attenuation of the angiotensin action will results in lowering of BP and improvement of protection against heart failure (Carson et al., (2001); Clin. Cardiol.; 24, 183-190).

In recent years, numerous orally active, selective ARA II antagonists have been synthesized and authorised by Competent Authorities for the treatment of Essential Hypertension, Heart Failure and Cardiovascular Prevention. Longer acting ARA II antagonists such as the sartans irbesartan, candesartan, and telmisartan may be more effective than shorter acting ARA II antagonists such losartan, providing better 24-hour control of BP. Moreover, they have better tolerability profile when compared with other blockers of the renin-angiotensin system such as ACE inhibitors. ARA II antagonists offer a number of benefits beyond those offered by the older classes of antihypertensive agents. These include specificity at a molecular level, virtually no side effects, and reversal of the remodelling that occurs in target organs damaged by the chronic effects of high BP.

Beta blockers (or β blockers) are drugs that block norepinephrine and epinephrine from binding to beta receptors. Beta-locker therapy plays a major role in the treatment of CVDs. For decades, Beta-blockers have been widely used in the treatment of hypertension and are still recommended as first-line drugs in hypertension guidelines. Moreover, after MI and in patients with HF, treatment with β blockers prevents re-infarction, hospitalisation for heart failure, and premature death (Freemantle et al., (1999), BMJ, 318: 1730-37; CIBIS-II, (1999), The cardiac insufficiency bisoprolol study II (CIBIS-II): a randomised trial, Lancet, 353: 9-13; MERIT-HF, (1999), Effect of metoprolol CR/XL in chronic heart failure: Metoprolol CR/XL Randomised Intervention Trial in Congestive heart failure (MERIT-HF), Lancet, 353: 2001-07; Packer et al., (2002), Circulation, 106: 2194-99).

Beta blockers can be broadly classified into (a) non-selective, those producing a competitive blockade of both b1- and b2-adrenergic receptors and (b) those with much higher affinity for the b1 than for the b2 receptors usually called b1-selective or cardio selective (in the context of this invention they are called selective β₁ receptor blockers). The non-cardioselective agents are more likely to produce adverse events, for example, they can cause airway constriction since beta-2 receptors are found in the airway. Metoprolol, atenolol and bisoprolol are examples of well-known cardioselective beta-blockers or in the context of this invention selective β₁ receptor blockers.

As said above, research has shown that monotherapy in this field of treatment had often not shown sufficient results. Accordingly, this invention started to focus on the use of combination therapy. It is understood that combination therapy could have many benefits. In terms of efficacy, using two complementary agents in combination (combination of mechanisms of action) could possibly result in greater efficacy than high-dose monotherapy (only one mechanism of action) if the right partners are chosen. Also in terms of safety, as far as side effects are concerned, the proper choice of combined compounds will possibly get similar or fewer side effects with complementary drugs used in combination than with high-dose monotherapy. Thus, by combining properly chosen compounds in combination therapy, efficacy and safety would go both in the same direction. Additionally, combination regimens will be associated with significantly better compliance and adherence to treatment, maximizing the long term efficacy of the treatment.

One interesting part of such a combination therapy was identified as being the sartan telmisartan.

### Telmisartan, 4'-[2-n-propyl-4-methyl-6-(1-methylbenzimid-azol-2-yl)-benzimidazol-1-ylmethyl]biphenyl-2-carboxylic acid

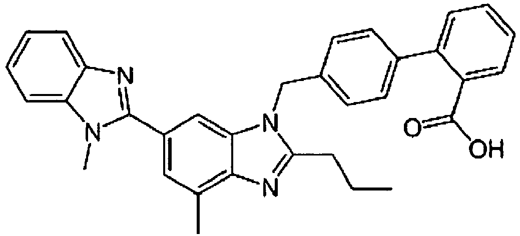

Telmisartan is an angiotensin AT1 antagonist and a PPARgamma modulator that was launched more than 15 years ago for the oral treatment of hypertension. Angiotensin II is the principal pressor agent of the renin-angiotensin system, with effects that include vasoconstriction, stimulation of synthesis and release of aldosterone, cardiac stimulation, and renal reabsorption of sodium. Telmisartan blocks the vasoconstrictor and aldosterone-secreting effects of angiotensin II by selectively blocking the binding of angiotensin II to the AT1 receptor in many tissues, such as vascular smooth muscle and the adrenal gland. Its action is therefore independent of the pathways for angiotensin II synthesis.

Telmisartan is practically insoluble in purified water (<0,1 mg/ml) but freely soluble (>100 mg/ml) in various organic solvents (96% ethanol, methanol, methylene chloride).

Starting from sartans like telmisartan, this invention identified beta blockers, especially selective β₁ receptor blockers, as the other group of agents that could form the other (or another) part of a successful combination therapy, or from which the combination partners of a successful combination therapy with a sartan like telmisartan could be selected.

Currently the association of ARA II antagonists and beta-blockers in a single pill is not available. This association will be of interest both in the management of hypertension per se as in the management of established HF and MI.

Combination of a sartan such as telmisartan and beta blockers (selective β₁ receptor blockers) offers complementary mechanisms of action for the management of hypertension, and it will be of special relevance in hypertension management in compelling indications for beta-blockers and ARA II antagonists (HF, MI).

The objective of this invention was to provide new means of improving the properties of sartans, especially telmisartan, especially in regard to the treatment of hypertension, and it will be of special relevance in hypertension management in compelling indications for beta-blockade and ARA II antagonism (heart failure and myocardial infarction), by providing new drugable forms of sartans like telmisartan. Especially desirable improvements/advantages of the new drugable form would include:
- improvement of physicochemical properties in order to facilitate the formulation, the manufacture, or to enhance the absorption and/or the bioavailability
- being more active when compared to the sartan like telmisartan
- providing a form of the sartan like telmisartan with a further beta blocker having a beneficial pharmacological effect in itself, thus allowing for a highly efficient dose/weight relation of the final active principle
- allowing the use of a lower therapeutic dose of either the sartan (telmisartan) and the further beta blocker or of both
- having a synergistic effect through the combination of the sartan like telmisartan and the further beta blocker in the same new drugable form
- being easily obtainable, easy to manufacture
- allowing more flexibility in formulating, or facilitating its formulation,
- being highly soluble, thus allowing better dissolution rates, especially if dissolving in an aqueous physiological surrounding
- improve stability of e.g. a co-crystal in comparison with the physical mixture at the same sartan (telmisartan) : beta blocker ratio
- allowing new routes of administration
- allowing to combine the sartan like telmisartan with a chemically usually non-compatible active agent in the same formulation or even in immediate contact, without having to isolate the sartan like telmisartan
- minimizing/reducing the side effects, especially the severe side effects, assigned to the sartan like telmisartan.

This objective was achieved by providing new crystalline forms of (comprising) a sartan (like telmisartan or candesartan or candesartan cilexitil) and beta blockers (selective β₁ receptor blockers). It was found that the sartans (like telmisartan or candesartan or candesartan cilexitil) was able to form co-crystals and salts with active beta blockers, especially with active selective β₁ receptor blockers. These new crystalline forms show improved properties if compared to the sartans (like telmisartan or candesartan or candesartan cilexitil) alone.

This key objective was achieved by providing new crystalline forms of (comprising) telmisartan and beta blockers (selective β₁ receptor blockers). It was found that telmisartan was able to form co-crystals and salts with active beta blockers, especially with active selective β₁ receptor blockers. These new crystalline forms show improved properties if compared to telmisartan alone.

The physico-chemical properties are improved. The formulation of the association is even easier with a solid to manipulate and an enhanced stability. The solubility may be also enhanced.

Another advantage is that the combination of two active principles into one unique species seems to allow for a better Pharmacokinetic/Pharmacodynamic (PKPD) which helps in the treatment of hypertension, heart failure and myocardial infarction.

Thus, the present invention is drawn to a new crystalline form comprising a sartan (like telmisartan or candesartan or candesartan cilexitil) either as a free acid or as a physiologically acceptable salt and at least one beta blocker either in free form or as a physiologically acceptable salt.

In its core, the present invention is drawn to a new crystalline form comprising telmisartan either as a free acid or as a physiologically acceptable salt and at least one beta blocker either in free form or as a physiologically acceptable salt.

"Sartan" as used herein is defined as longer acting ARA II antagonist and especially encompasses irbesartan, candesartan (or candesartan cilexitil), and telmisartan.

"Free form" as used herein is defined as either a free acid or a free base.

"Beta blocker" as used herein is defined as already set-out above and are drugs that block norepinephrine and epinephrine from binding to beta receptors. This invention is mainly focused on selective β₁ receptor blocker, active agents (beta blockers) that have a higher affinity for the β₁ receptor than for the β₂ receptor. Known selective β₁ receptor blockers are acebutolol, atenolol, betaxolol, bisoprolol, celiprolol, esmolol, metoprolol, or nebivolol.

The "crystalline form" may either be a salt of a co-crystal.

"Salt" as used herein is defined as a crystalline material comprising two or more compounds at ambient temperature (20 to 25°C, preferably 20°C), of which at least two are held together by ionic interaction.

"Co-crystal" as used herein is defined as a crystalline material comprising two or more compounds at ambient temperature (20 to 25°C, preferably 20°C), of which at least two are held together by weak interaction, wherein at least one of the compounds (in this case the beta blocker) is a co-crystal former. Weak interaction is being defined as an interaction which is neither ionic nor covalent and includes for example: hydrogen bonds, van der Waals forces, and π-π-interactions. The co-crystals may include one or more solvate molecules (e.g. water, alcohol or acid) in the crystalline lattice. Just for the sake of clarity the distinction between crystalline salt and a co-crystal has to be stressed here. An API bound to another compound forming a salt by means of ionic interaction can be considered as one "compound" according to the invention, but it cannot be considered as two compounds by itself.

In scientific literature there currently is some discussion on the proper use of the word co-crystal (see for example Desiraju, Cryst. Eng. Comm., 2003, 5(82), 466-467 and Dunitz, CrystEngComm, 2003, 5(91), 506). A recent article by Zaworotko (Zaworotko, Crystal Growth & Design, Vol. 7, No. 1, 2007, 4-9) gives a definition of co-crystal which is in line with the definition given above.

In one embodiment of the crystalline form according to the invention the at least one beta blocker is a selective β₁ receptor blocker. Preferably the at least one selective β₁ receptor blocker is selected from acebutolol, atenolol, betaxolol, bisoprolol, celiprolol, esmolol, metoprolol, or nebivolol, preferably is selected from metoprolol, atenolol, nebivolol or bisoprolol, more preferably is selected from metoprolol, atenolol, or bisoprolol.

### Metoprolol, 1-(4-(2-methoxyethyl)phenoxy)-3-(isopropylamino)-propan-2-ol

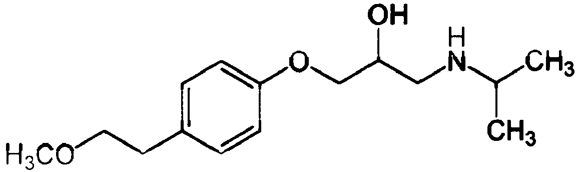

Metoprolol is a beta1-selective adrenoceptor blocking agent used in treatment of several diseases of the cardiovascular system, especially hypertension. This preferential effect is not absolute, however, and at higher plasma concentrations, metoprolol also inhibits beta2-adrenoreceptors, chiefly located in the bronchial and vascular musculature.

### Atenolol, 4-[2-hydroxy-3-[(1-methylethyl)amino]propoxy]benzeneacetamide

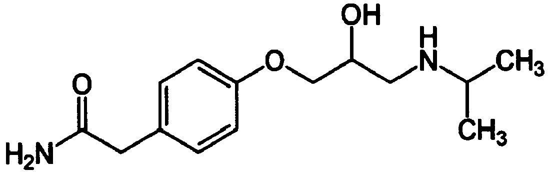

Atenolol, first launched in 1975, is a synthetic, beta1-selective adrenoceptor blocking agent that is commercialized by AstraZeneca for the oral treatment of angina pectoris, arrhythmia, hypertension and for intravenous use as early intervention in acute myocardial infarction. The drug is also available for the management of moderate to severe congestive heart failure (CHF).

### Bisoprolol hemifumarate {1-{4-[(2-isopropoxyethoxy)methyl]phenoxy}-3-(isopropylamino)propan-2-ol}₂ fumarate

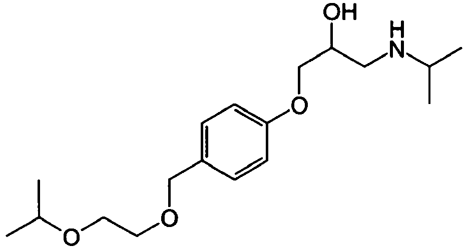

Bisoprolol fumarate's most prominent activity is a negative chronotropic effect, resulting in a reduction in resting and exercise heart rate. There is a fall in resting and exercise cardiac output with little observed change in stroke volume, and only a small increase in right atrial pressure, or pulmonary capillary wedge pressure at rest or during exercise. The mechanism of action of its antihypertensive effects has not been completely established. Factors which may be involved include antagonism of beta-adrenoceptors, inhibition of renin release by the kidneys and diminution of tonic sympathetic outflow from the vasomotor centers in the brain.

In a further embodiment of the crystalline form according to the invention the crystalline form comprises in addition to telmisartan and the at least one beta blocker an additional compound selected from alcohol, acid or water, preferably selected from ethanol, isopropanol, fumaric acid or water; more preferably selected from ethanol, fumaric acid or water.

In a further embodiment of the crystalline form according to the invention the ratio between the sartan like telmisartan and the at least one beta blocker is chosen in such a way that if compared to either the sartan like telmisartan alone and/or to a mixture of telmisartan and the at least one beta blocker
- the solubility of the crystalline form is increased; and/or
- the dose response of the crystalline form is increased; and/or
- the efficacy of the crystalline form is increased; and/or
- the dissolution of the crystalline form is increased; and/or
- the bioavailability of the crystalline form is increased; and/or
- the stability of the crystalline form is increased; and/or
- the hygroscopicity of the crystalline form is decreased; and/or
- the form diversity of the crystalline form is decreased; and/or
- the morphology of the crystalline form is modulated.

In one embodiment of the crystalline form according to the invention the crystalline form is a salt. Preferably the salt is selected from a salt of candesartan cilexitil and nebivolol, telmisartan and metoprolol, or a salt of telmisartan and atenolol, more preferably the salt is selected from a salt of telmisartan and metoprolol, or a salt of telmisartan and atenolol, most preferably is selected from a salt telmisartan - metoprolol (1:1), or a salt telmisartan - atenolol - H₂O (1:1:1).

One embodiment of the crystalline form according to the invention in which the crystalline form is a salt relates to a salt of telmisartan - metoprolol (1:1)
whose the endothermic sharp peak corresponding to the melting point has an onset at 131 °C, preferably at 130.9 °C, most preferably at 130.92 °C; and/or
which shows an X-Ray powder diffraction pattern with peaks [2θ] at 6.84, 13.15, 20.06, 20.36, and 22.71° or at 6.8, 13.2, 20.1, 20.4, and 22.7°, preferably with peaks [2θ] at 6.84, 10.06, 13.15, 16.49, 18.07, 20.06, 20.36, 22.56, and 22.71°; more preferably with peaks [2θ] at 6.04, 6.84, 10.06, 13.15, 13.96, 14.35, 16.15, 16.49, 17.06, 17.76, 18.07, 19.00, 20.06, 20.36, 21.44, 21.65, 22.56, 22.71, 23.53, and 25.12°; most preferably with peaks [2θ] at 6.04, 6.84, 8.46, 10.06, 10.58, 11.74, 12.12, 13.15, 13.71, 13.96, 14.35, 14.60, 15.03, 15.38, 16.15, 16.49, 17.06, 17.76, 18.07, 19.00, 20.06, 20.36, 20.55, 21.25, 21.44, 21.65, 22.56, 22.71, 23.01, 23.53, 24.29, 24.56, 24.91, 25.12, 26.09, 26.63, 27.16, 29.28, 29.89, 30.25, 33.11, 33.72, and 38.19.

One further embodiment of the crystalline form according to the invention in which the crystalline form is a salt relates to a salt of telmisartan - atenolol - H₂O (1:1:1)
whose endothermic sharp peak corresponding to the melting point has an onset at 113 °C, preferably at 113.1 °C, most preferably at 113.05 °C; and/or
which shows an X-Ray powder diffraction pattern with peaks [2θ] at 8.45, 14.67, 17.86, and 19.66 °, or at 8.5, 14.7, 17.9, and 19.7 °, or at 8.45, 14.67, 15.99, 17.86, 19.66, 20.99, and 22.19 °; preferably with peaks [2θ] at 8.45, 14.43, 14.67, 15.99, 17.02, 17.86, 19.14, 19.66, 20.99, 22.19, 25.48, and 25.66 °; more preferably with peaks [2θ] at 8.45, 8.68, 11.95, 12.44, 14.43, 14.67, 15.48, 15.62, 15.99, 17.02, 17.86, 18.07, 19.14, 19.66, 20.56, 20.99, 22.19, 23.48, 25.48, 25.66, and 25.82, °; most preferably with peaks [2θ] at 8.45, 8.68, 9.34, 10.35, 10.60, 11.95, 12.44, 14.43, 14.67, 15.48, 15.62, 15.99, 17.02, 17.47, 17.86, 18.07, 18.81, 19.14, 19.66, 20.56, 20.99, 21.71, 22.19, 22.58, 23.48, 23.80, 24.11, 24.47, 24.90, 25.48, 25.66, 25.82, 26.64, 27.26, 27.89, 28.43, 29.10, 29.76, 30.69, 31.09, 32.16, 34.43, 38.49, and 38.97 °; and/or
which has a monoclinic unit cell with the following dimensions:
a = 22.76 Å,
b = 9.24 Å, and
c = 22.18 Å,
with an angle β of 113.98 °
or which has a monoclinic unit cell with the following dimensions:
a = 22.7566 Å,
b = 9.2383 Å, and
c = 22.1794 Å,
with an angle β of 113.980 °

In another embodiment of the crystalline form according to the invention the crystalline form is a co-crystal. Preferably the co-crystal is selected from a co-crystal comprising telmisartan and atenolol or a co-crystal comprising telmisartan and bisoprolol, more preferably is selected from a co-crystal of telmisartan - atenolol - EtOH (6:3:2), a co-crystal of telmisartan - bisoprolol - fumaric acid (2:1:1), or a co-crystal of telmisartan - bisoprotol - fumaric acid (4:1:2).

One embodiment of the crystalline form according to the invention in which the crystalline form is a co-crystal relates to a co-crystal of telmisartan - atenolol - EtOH (6:3:2)
whose endothermic sharp peak corresponding to the melting point has an onset at 194 °C, preferably at 194.5 °C, most preferably at 194.49 °C; and/or
which shows an X-Ray powder diffraction pattern with peaks [2θ] at 6.6, 10.7, and 15.7° or at 6.64, 10.67, and 15.66°, or at 6.64, 9.75, 10.67, 15.66, 18.15, 18.49, 20.60, and 20.85 °; preferably with peaks [2θ] at 6.64, 9.75, 10.67, 15.16, 15.66, 17.03, 18.15, 18.49, 19.29, 20.60, and 20.85°; more preferably with peaks [2θ] at 6.09, 6.64, 9.75, 10.67, 13.26, 13.87, 14.68, 15.16, 15.66, 17.03, 18.15, 18.49, 19.29, 20.60, 20.85, 22.64, 23.95, and 25.65°; most preferably with peaks [2θ] at 4.51, 6.09, 6.64, 9.75, 10.67, 11.53, 12.05, 12.66, 13.26, 13.87, 14.68, 15.16, 15.66, 17.03, 17.24, 18.15, 18.49, 19.29, 20.60, 20.85, 21.95, 22.64, 23.35, 23.95, 24.35, 24.77, 25.15, 25.65, 26.12, 26.82, 27.50, 28.60, 31.55, and 33.23°.

One further embodiment of the crystalline form according to the invention in which the crystalline form is a co-crystal relates to a co-crystal of telmisartan - bisoprolol - fumaric acid (2:1:1)
whose endothermic peak corresponding to the co-crystal transformation has an onset at 80 °C, preferably at 80.2 °C, most preferably at 80.17 °C; and/or
whose endothermic peak corresponding to the melting point has an onset at 148 °C, preferably at 147.5 °C; and/or
which shows an X-Ray powder diffraction pattern with peaks [2θ] at 6.2, 15.8, and 22.6 ° or at 6.17, 15.75, and 22.55 °; preferably with peaks [2θ] at 6.17, 14.94, 15.75, and 22.55 °; more preferably with peaks [2θ] at 6.17, 11.21, 14.94, 15.75, 20.10, 21.97, and 22.55 °; most preferably with peaks [2θ] at 6.17, 7.45, 8.93, 11.21, 13.39, 14.94, 15.75, 17.24, 18.56, 19.12, 20.10, 21.97, 22.55, 25.08, and 27.09 °.

One further embodiment of the crystalline form according to the invention in which the crystalline form is a co-crystal relates to a co-crystal of telmisartan - bisoprolol - fumaric acid (4:1:2)
whose endothermic peak corresponding to the co-crystal degradation has an onset at 159 °C, preferably at 158.7 °C, most preferably at 158.69 °C; and/or
whose the endothermic peak corresponding to the melting point has an onset at 247 °C, preferably at 246.9 °C, most preferably at 246.92 °C; and/or
which shows an X-Ray powder diffraction pattern with peaks [2θ] at 6.2, 15.8, and 22.0 °, or at 6.18, 15.79, and 21.96 °; preferably with peaks [2θ] at 6.18, 7.47, 15.79, 20.10, 21.96, and 22.51 °; more preferably with peaks [2θ] at 6.18, 7.47, 11.24, 13.39, 14.98, 15.79, 18.59, 19.15, 20.10, 21.96, and 22.51 °; most preferably with peaks [2θ] at 6.18, 7.47, 8.85, 11.24, 12.13, 13.39, 14.98, 15.79, 17.22, 18.59, 19.15, 19.48, 20.10, 20.49, 21.96, 22.51, 24.37, 25.10, 27.27, and 27.62 °.

One other embodiment of the invention is a crystalline form of the sartan candensartan cilexetil - with nebivolol in which the crystalline form is a salt, preferably relating to a salt of candensartan cilexetil - nebivolol (1:1)
whose endothermic peak corresponding to the melting point has an onset at 95 °C, preferably at 94.8 °C, most preferably at 94.83 °C; and/or
which shows an X-Ray powder diffraction pattern with peaks [2θ] at 10.1, and 10.5 ° or at 10.11, and 10.53 °; preferably with peaks [2θ] at 10.11, 10.53, 15.97, 21.69, 23.31, and 24.28°; more preferably with peaks [2θ] at 10.11, 10.53, 13.62, 15.41, 15.97, 16.25, 17.34, 18.54, 20.26, 21.11, 21.69, 23.31, and 24.28°; most preferably with peaks [2θ] at 6.07, 8.42, 10.11, 10.53, 12.82, 13.62, 15.41, 15.97, 16.25, 16.99, 17.34, 18.54, 19.02, 19.68, 20.26, 21.11, 21.69, 22.47, 23.31, 24.28, 24.97, 26.82, 27.78, 29.05, 32.13, and 37.73°.

Another aspect of the present invention relates to a process for the production of a crystalline form according to the invention as described above comprising the steps of:
(a) dissolving or suspending the sartan or telmisartan and at least one beta blocker in a solvent or mixture of solvents,
(b) optionally mixing or stirring the solution or suspension,
(c) optionally before, during or after step (b) keeping the mixed solution/suspension at ambient temperature or above;
(d) optionally filtering-off and/or washing the resulting solid with a solvent, and
(e) drying the solid optionally at ambient temperature and/or vacuum.

"Ambient temperature" (also known as "room temperature") is defined here as a temperature between 20 and 25°C, preferably being 20°C.

In a preferred embodiment the organic solvent used in step a) in this process is selected from acetone, iBuOAc (=isobutyl acetate), ethanol, water, acetonitrile, chloroform, isopropanol, ethanol, toluene, ethyl acetate, acetone, heptane, or a mixture of acetone and heptane.

The active principle sartan or telmisartan that forms part of the co-crystal according to the invention is a well-known drug as are the beta blockers comprised in the crystalline form. Due to this, a further object of the present invention is a medicament comprising at least one crystalline form according to the invention and optionally one or more pharmaceutically acceptable excipients. Thus the invention also relates to a medicament comprising at least one crystalline form (either co-crystal or salt) according to the invention as described above.

The invention also relates to a pharmaceutical composition comprising a therapeutically effective amount of the crystalline form (either co-crystal or salt) according to the invention as described above in a physiologically acceptable medium. The invention also relates to a pharmaceutical composition comprising the crystalline form (either co-crystal or salt) according to the invention and optionally one or more pharmaceutically acceptable excipients.

The invention also relates to a pharmaceutical composition comprising a therapeutically effective amount of the crystalline form (either co-crystal or salt) according to the invention and optionally one or more pharmaceutically acceptable excipients.

The invention also concerns a medicament or pharmaceutical composition comprising (preferably a therapeutically effective amount of) at least one crystalline form (either co-crystal or salt) according to the invention as described above and optionally one or more pharmaceutically acceptable excipients for use in the treatment of hypertension, heart failure and myocardial infarction.

In general, in most embodiments in which the co-crystals according to the invention are used (e.g. treatment of hypertension, heart failure and myocardial infarction) these crystalline forms would be formulated into a convenient pharmaceutical formulation or a medicament.

Accordingly a desirable advantage of a crystalline form (either a co-crystal or a salt) according to the invention would be showing improved pharmaceutical properties and features for use in such a formulation, especially when compared to a sartan like telmisartan either as a free acid or as a physiologically acceptable salt alone. Thus, the crystalline form according to the invention should desirably show at least one, preferably more, of the following features - especially if used or for use in a convenient pharmaceutical formulation or a medicament:
- to have a very small particle size, e.g. from 300 µm or lower; or
- to be and/or remain essentially free of agglomerates; or
- to be less or not very hygroscopic; or
- to help in formulating controlled release or immediate release formulations; or
- to have a high chemical stability; or
if given to a patient
- to decrease the inter- and intra-subject variability in blood levels; or
- to show a good absorption rate (e.g. increases in plasma levels or AUC); or
- to show a high maximum plasma concentration (e.g. Cₘₐₓ); or
- to show decreased time to peak drug concentrations in plasma (tₘₐₓ); or
- to show changes in half life of the compound (t_{1/2}), in whichever direction this change is preferably directed.

The medicament or pharmaceutical compositions according to the present invention may be in any form suitable for the application to humans and/or animals, preferably humans including infants, children and adults and can be produced by standard procedures known to those skilled in the art. The medicament of the present invention may for example be administered parenterally, including intramuscular, intraperitoneal, or intravenous injection, transmucosal or sublingual application; or orally, including administration as tablets, pellets, granules, capsules, lozenges, aqueous or oily solutions, suspensions, emulsions, sprays or as reconstituted dry powdered form with a liquid medium.

Typically, the medicaments according to the present invention may contain 1-60 % by weight of one or more of the crystalline form (either co-crystal or salt) as defined herein and 40-99 % by weight of one or more pharmaceutically acceptable excipients.

The compositions of the present invention may also be administered topically or via a suppository.

The daily dosage for humans and animals (especially adults) may vary depending on factors that have their basis in the respective species or other factors, such as age, sex, weight or degree of illness and so forth. The daily dosage for humans preferably is in the range of 1 to 500 milligrams of the sartan or especially telmisartan (or the equivalent in the crystalline form (either a co-crystal or a salt)) or preferably 5 to 250 milligrams of the sartan or especially telmisartan (or the equivalent in the crystalline form (either a co-crystal or a salt)) or more preferably 5 to 100 or 20 to 80 milligrams of the sartan or especially telmisartan (or the equivalent in the crystalline form (either a co-crystal or a salt)). This can be administered during one or several intakes per day, preferably once or twice daily e.g. in a dosage of 20, 40 or 80 mg of telmisartan (or another sartan) or the equivalent in the crystalline form (either a co-crystal or a salt).

The daily dosage for humans is in the range of 5 to 1500 mg of crystalline form (either a co-crystal or a salt) according to the invention or of the respective part of the co-crystal according to the invention, preferably is in the range of 5 to 500 milligrams, more preferably in the range of 10 to 300 milligrams, most preferably in the range of 20 to 200 milligrams preferably to be administered during one or several (preferably one or two) intakes per day.

A further aspect of the invention relates to the use of a crystalline form (either a co-crystal or a salt) according to the invention as described above for the treatment of hypertension, heart failure or myocardial infarction. Preferably this use is provided in the form of a medicament or a pharmaceutical composition according to the invention as described above.

Another aspect of the invention relates to the use of a crystalline form (either a co-crystal or a salt) according to the invention as described above in the manufacture of a medicament for the treatment of hypertension, heart failure or myocardial infarction.

The invention also relates to a crystalline form (either a co-crystal or a salt) according to the invention as described above for use in the treatment of hypertension, heart failure or myocardial infarction.

Another object of the current invention is a method of treatment of hypertension, heart failure or myocardial infarction by providing to a patient in need thereof a sufficient amount of a crystalline form (either a co-crystal or a salt) according to the invention as described above. Preferably the crystalline form (either a co-crystal or a salt) according to the invention is provided in physiologically suitable form like e.g. in the form of a medicament or a pharmaceutical composition according to the invention as described above e.g. comprising 10 to 100 or 20 to 80 milligrams of the sartan or especially telmisartan (or the equivalent in the crystalline form (either a co-crystal or a salt)).

The present invention is illustrated below with the help of the following figures and examples. These illustrations are given solely by way of example and do not limit the invention.

### Brief description of the figures:

**Figure 1****:** Differential scanning calorimetry (DSC) analysis of telmisartan - metropolol (1:1) salt
**Figure 2****:** Thermogravimetric analysis (TGA) of telmisartan - metropolol (1:1) salt
**Figure 3****:** X-ray powder diffraction (XRPD) pattern of telmisartan - metropolol (1:1) salt
**Figure 4****:** Differential scanning calorimetry (DSC) analysis of telmisartan - atenolol - EtOH (6:3:2) co-crystal
**Figure 5****:** Thermogravimetric analysis (TGA) of telmisartan - atenolol - EtOH (6:3:2) co-crystal
**Figure 6****:** X-ray powder diffraction (XRPD) pattern of telmisartan - atenolol - EtOH (6:3:2) co-crystal
**Figure 7****:** Differential scanning calorimetry (DSC) of telmisartan - atenolol - H₂O (1:1:1) salt
**Figure 8****:** Thermogravimetric analysis (TGA) of telmisartan - atenolol - H₂O (1:1:1) salt
**Figure 9****:** X-ray powder diffraction (XRPD) pattern of telmisartan - atenolol - H₂O (1:1:1) salt
**Figure 10****:** Crystal structure of telmisartan - atenolol - H₂O (1:1:1) salt
**Figure 11****:** Differential scanning calorimetry (DSC) of telmisartan- bisoprolol - fumaric acid (2:1:1) co-crystal
**Figure 12****:** Thermogravimetric analysis (TGA) of telmisartan- bisoprolol - fumaric acid (2:1:1) co-crystal
**Figure 13****:** X-ray powder diffraction (XRPD) pattern of telmisartan- bisoprolol - fumaric acid (2:1:1) co-crystal
**Figure 14****:** Differential scanning calorimetry (DSC) of telmisartan - bisoprolol - fumaric acid (4:2:1) co-crystal
**Figure 15****:** Thermogravimetric analysis (TGA) of telmisartan - bisoprolol - fumaric acid (4:2:1) co-crystal
**Figure 16****:** X-ray powder diffraction (XRPD) pattern of telmisartan - bisoprolol - fumaric acid (4:2:1) co-crystal
**Figure 17****:** Differential scanning calorimetry (DSC) of candensartan cilexetil - nebivolol (1:1) (salt)
**Figure 18****:** Thermogravimetric analysis (TGA) of candensartan cilexetil - nebivolol (1:1) (salt)
**Figure 19****:** X-ray powder diffraction (XRPD) pattern of candensartan cilexetil - nebivolol (1:1) (salt)

### EXAMPLES

### Example 1: Telmisartan - Metropolol (1:1) (salt)

### Example 1.1: Slurrying in Acetone

To an assay tube equipped with magnetic stirrer containing metoprolol (24 mg, 0.09 mmol, 2.0 eq) and telmisartan (25 mg, 0.048 mmol, 1 eq), was added acetone (0.1 mL). The resultant suspension was stirred at room temperature for 6 hours. The solid was filtered with a sintered funnel (porosity 4), washed with acetone (0.2 mL) and dried under vacuum at room temperature to give salt of telmisartan - metoprolol (1:1) as a white solid.

### Example 1.2: Slurrying in iBuOAc (750 mg scale-up)

To an assay tube equipped with magnetic stirrer containing metoprolol (400 mg, 1.496 mmol, 1.48 eq) and telmisartan (520.0 mg, 1.010 mmol), was added iBuOAc (1.2 mL). The resultant suspension was seeded with form obtained in the Example 1.1 and stirred at room temperature for 20 hours. The solid was filtered with a sintered funnel (porosity 4), washed with AcOiBu (2 x 1.7 mL) and dried under vacuum at room temperature to give salt of telmisartan - metropolol (1:1) as a white solid. (745.8 mg, 94% yield).

### ¹H NMR

Proton Nuclear Magnetic Resonance analyses were recorded in deuterated chloroform (CDCl₃) in a Varian Mercury 400 spectrometer, equipped with a broadband probe ATB 1 H/19F/X of 5 mm. Spectra were acquired dissolving 5-10 mg of sample in 0.6 mL of deuterated solvent.

¹H NMR spectrum in deuterated chloroform (CDCl₃) at 400 MHz shows peaks at 8.16-8.11 (m, 1H); 7.88 (dd, *J* = 1.6 Hz, *J* = 7.4 Hz, 1H ); 7.41 (dq, *J* = 1.6 Hz, *J* = 7.4 Hz, 1H) 7.40-7.29 (m, 7H); 7.15 (s, 2H); 7.13-7.10 (m, 4H); 6.80-6.76 (m, 2H); 5.39 (s, 2H); 4.14 - 4.06 (m, 1H); 3.91 (dd, *J* = 5.1 Hz, *J* = 9.4 Hz, 1H); 3.83 (dd, *J* = 5.5 Hz, *J* = 9.4 Hz, 1H); 3.76 (s, 3H); 3.55 (t, *J* = 7.0 Hz, 2H); 3.34 (s, 3H); 3.09-3.03 (m, 2H); 2.96 - 2.88 (m, 2H); 2.81 (t, *J* = 7.0 Hz, 2H); 2.76 - 2.70 (m, 4H); 1.96 (sx, *J* = 7.4 Hz, 2H); 1.12 (t, *J* = 7.4 Hz, 3H); 1.02 (d, *J* = 6.4 Hz, 6H) ppm.

### IR

FTIR spectra were recorded using a Thermo Nicolet Nexus 870 FT-IR, equipped with a beamsplitter KBr system, a 35 mW He-Ne laser as the excitation source and a DTGS KBr detector. The spectra were acquired in 32 scans at a resolution of 4 cm⁻¹.

The sample (KBr pellet) shows a Fourier Transform Infra Red spectrum with absorption bands at 2933.6 (m), 1612.0 (m), 1513.3 (s), 1451.3 (s), 1393.1 (s), 1322.5 (m), 1279.8 (m), 1243.4 (s), 1180.4 (m), 1159.4 (m), 1108.5 (m), 1083.6 (m), 1040.1 (m), 1005.4 (m), 966.9 (m), 947.1 (m), 920.7 (m), 849.6 (m), 829.3 (m), 765.0 (m), 743.6 (s), 712.1 (m), 657.7 (m), 549.8 (m), 531.3 (m) cm⁻¹.

### DSC

DSC analyses were recorded with a Mettler DSC822^{e}. A sample of 1.1700 mg was weighed into 40 µL aluminium crucible with a pinhole lid and was heated, under nitrogen (50 mL/min), at 10°C/min from 30 to 300 °C.

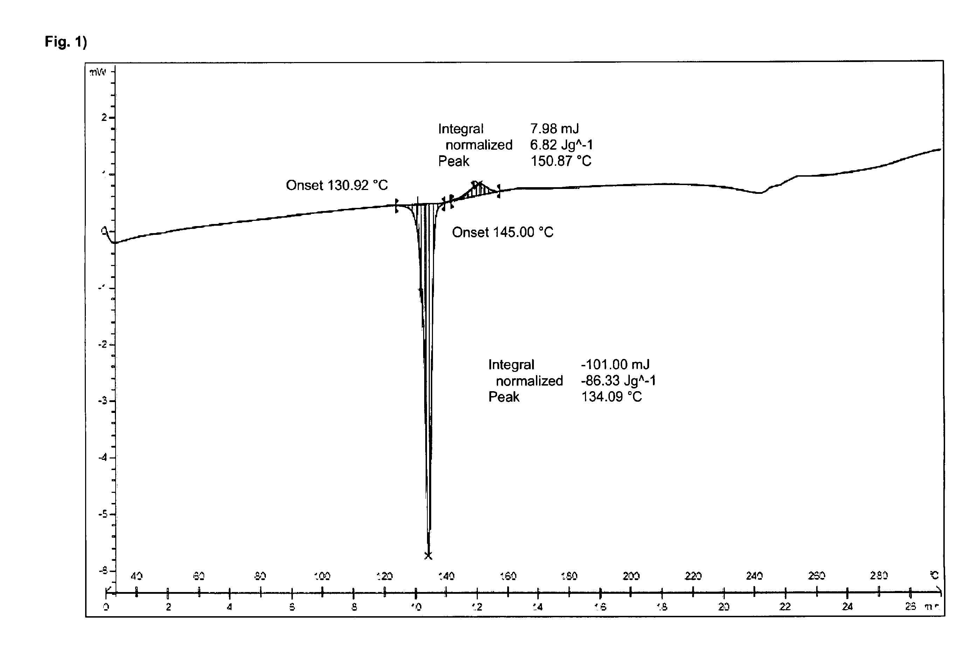

The novel type of crystal of the present invention is characterized in that the endothermic sharp peak corresponding to the melting point has an onset at 130.92 °C (fusion enthalpy - 86.33 J/g), measured by DSC analysis (10 °C/min) (see figure 1).

### TGA

Thermogravimetric analyses were recorded in a thermogravimetric analyzer Mettler TGA/SDTA851^{e}. A sample of 4.6956 mg was weighed into a 70 µL alumina crucible with a pinhole lid and was heated at 10 °C/min from 30 to 300 °C, under nitrogen (50 mL/min).

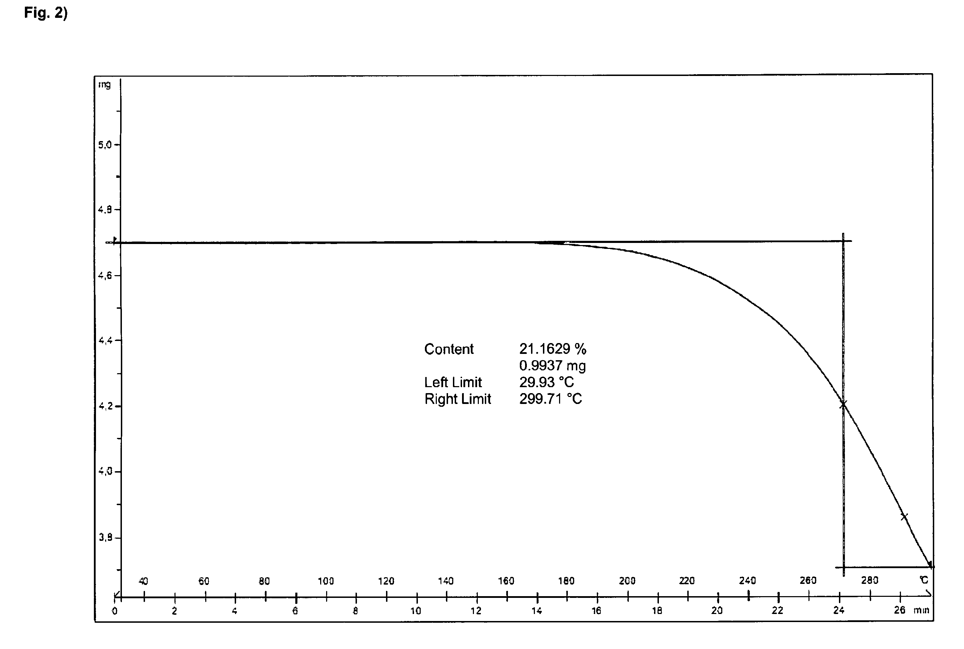

The TG analysis of the crystalline form according to the invention shows no weight loss between 30 and 170 °C (see figure 2).

### XRPD: X-ray powder diffraction pattern

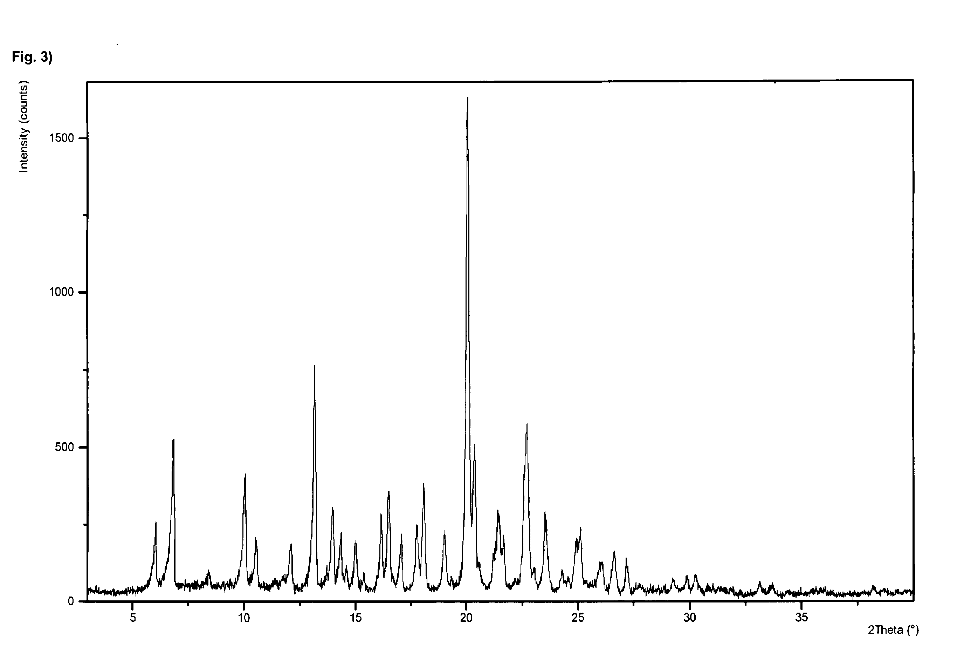

XRPD analysis was performed using a Philips X'Pert diffractometer with Cu K_{α} radiation in Bragg-Brentano geometry. The system is equipped with a monodimensional, real time multiple strip detector. The diffractogram was recorded from 3° to 40° (2θ) at a scan rate of 17.6° per minute (see figure 3).

List of selected peaks (only peaks with relative intensity greater than or equal to 1 % are indicated):

| **2θ (°)** | **d (A)** | **I (%)** | **2θ (°)** | **d (A)** | **I (%)** |
|---|---|---|---|---|---|
| 6.04 | 14.63 | 13 | 20.55 | 4.32 | 7 |
| 6.84 | 12.92 | 29 | 21.25 | 4.18 | 9 |
| 8.46 | 10.46 | 2 | 21.44 | 4.14 | 14 |
| 10.06 | 8.79 | 21 | 21.65 | 4.10 | 10 |
| 10.58 | 8.36 | 8 | 22.56 | 3.94 | 19 |
| 11.74 | 7.54 | 2 | 22.71 | 3.92 | 33 |
| 12.12 | 7.30 | 7 | 23.01 | 3.86 | 5 |
| 13.15 | 6.73 | 41 | 23.53 | 3.78 | 15 |
| 13.71 | 6.46 | 4 | 24.29 | 3.66 | 3 |
| 13.96 | 6.34 | 15 | 24.56 | 3.62 | 2 |
| 14.35 | 6.17 | 10 | 24.91 | 3.57 | 9 |
| 14.60 | 6.07 | 3 | 25.12 | 3.55 | 10 |
| 15.03 | 5.89 | 9 | 26.09 | 3.42 | 5 |
| 15.38 | 5.76 | 4 | 26.63 | 3.35 | 8 |
| 16.15 | 5.49 | 14 | 27.16 | 3.28 | 6 |
| 16.49 | 5.38 | 19 | 29.28 | 3.05 | 2 |
| 17.06 | 5.20 | 11 | 29.89 | 2.99 | 3 |
| 17.76 | 4.99 | 12 | 30.25 | 2.95 | 3 |
| 18.07 | 4.91 | 21 | 33.11 | 2.71 | 2 |
| 19.00 | 4.67 | 11 | 33.72 | 2.66 | 2 |
| 20.06 | 4.43 | 100 | 38.19 | 2.36 | 1 |
| 20.36 | 4.36 | 26 | | | |

### Example 2: Telmisartan - Atenolol - EtOH (6:3:2) (co-crystal)

### Example 2.1: Slurrying in ethanol

To an assay tube equipped with magnetic stirrer containing atenolol (20 mg, 0.075 mmol) and telmisartan (40 mg, 0.075 mmol, 1 eq), was added ethanol (0.5 mL). The resultant slurring was stirred at room temperature for 15 hours. The solid was centrifuged and dried under vacuum at room temperature to give co-crystal of telmisartan - atenolol - ethanol (6:3:2) as a white solid (51 mg, 80% yield).

### Example 2.2: Slurrying in ethanol (500 mg scale)

To a round-bottom flask equipped with magnetic stirrer containing atenolol (115 mg, 0.43 mmol) and telmisartan (399 mg, 0.77 mmol, 1.8 eq), was added ethanol (2 mL). The resultant slurring was seeded with form obtained in Example 2.1 and stirred at room temperature for 24 hours with periodic sonication. The solid was filtered with a sintered funnel (porosity 4), washed with ethanol (3 x 0.2 mL) and dried under vacuum at room temperature to give co-crystal of telmisartan - atenolol - ethanol (6:3:2) as a white solid (448 mg, 79% yield).

### ¹H NMR

Proton nuclear magnetic resonance analyses were recorded in deuterated dimethyl sulphoxide (DMSO) in a Varian Mercury 400 spectrometer, equipped with a broadband probe ATB 1 H/19F/X of 5 mm. Spectra were acquired dissolving 5-10 mg of sample in 0.6 mL of deuterated solvent.

¹H NMR (DMSO, 400 MHz): δ = 7.72 (s, 1H); 7.68-7.64 (m, 1H); 7.57 (d, *J* = 7.2 Hz, 2H); 7.47 (s, 1H); 7.42 (dt, *J* = 1.2 Hz, *J* = 7.2 Hz, 1H); 7.39-7.31 (m, 3H); 7.27 (d, *J* = 7.2 Hz, 1.5H); 7.24 (dd, *J* = 1.6 Hz, *J* = 2.3 Hz, 0.5H); 7.21 (dd, *J* = 1.2 Hz, *J* = 7.4 Hz, 0.5H); 7.15 (dd, *J* = 8.4 Hz and 2.0 Hz, 3H); 6.84 (m, 1H); 6.83-6.77 (s br, 0.5H); 5.60 (s, 2H); 4.02-3.94 (m, 1H); 3.91-3.94 (m, 1H); 3.82 (s, 3H); 3.44 (q, *J* = 7.0 Hz, 0.66H), 3.28 (s, 2H); 2.96-2.89 (m, 2H); 2.84 (dd, *J* = 3.5 Hz, *J* = 12.1 Hz, 0.5H); 2.71 (dd, *J* = 8.2 Hz, *J* = 12.1 Hz, 0.5H); 2.63 (s, 3H); 1.83 (sx, *J* = 7.0 Hz, 2H), 1.05 (t, *J* = 7.0 Hz, 1 H), 1.02-0.98 (m, 6H).

### IR

FTIR spectra were recorded using a Thermo Nicolet Nexus 870 FT-IR, equipped with a beamsplitter KBr system, a 35mW He-Ne laser as the excitation source and a DTGS KBr detector. The spectra were acquired in 32 scans at a resolution of 4 cm⁻¹.

IR (KBr): v = 3401.5.8 (s), 3055.8 (s), 2966.1 (m), 2873.6 (s), 1683.4 (m), 1612.9 (s), 1532.2 (vs), 1511.4 (m), 1453.8 (m), 1390.7 (w), 1283.8 (s), 1245.5 (m), 1187.5 (vs), 1090.5 (vs), 1093.5 (m), 1006.1 (vs), 842.6 (vs), 743.4 (s) cm⁻¹.

### DSC

DSC analyses were recorded with a Mettler DSC822^{e}. A sample of 2.1030 mg was weighed into 40 µL aluminium crucible with a pinhole lid and was heated, under nitrogen (50 mL/min), at 10°C/min from 30 to 300 °C.

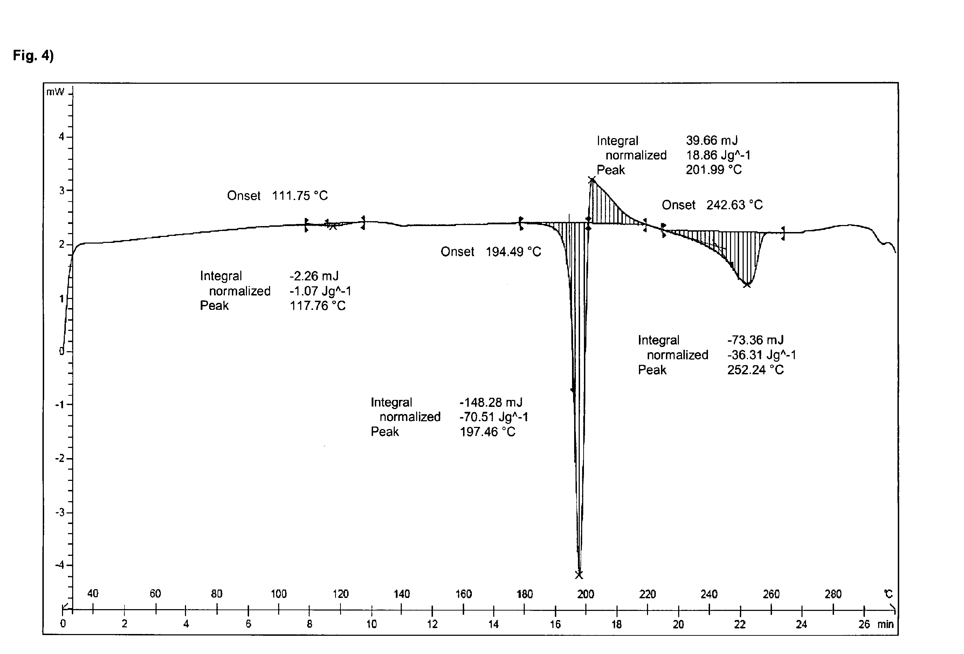

The novel type of crystal of the present invention is characterized in that the endothermic sharp peak corresponding to the melting point has an onset at 194.49 °C (fusion enthalpy - 70.51 J/g), measured by DSC analysis (10 °C/min) (see figure 4).

### TG

Thermogravimetric analyses were recorded in a thermogravimetric analyzer Mettler TGA/SDTA851^{e}. A sample of 3.6307 mg was weighed into a 70 µL alumina crucible with a pinhole lid and was heated at 10°C/min from 30 to 300°C, under nitrogen (50 mL/min).

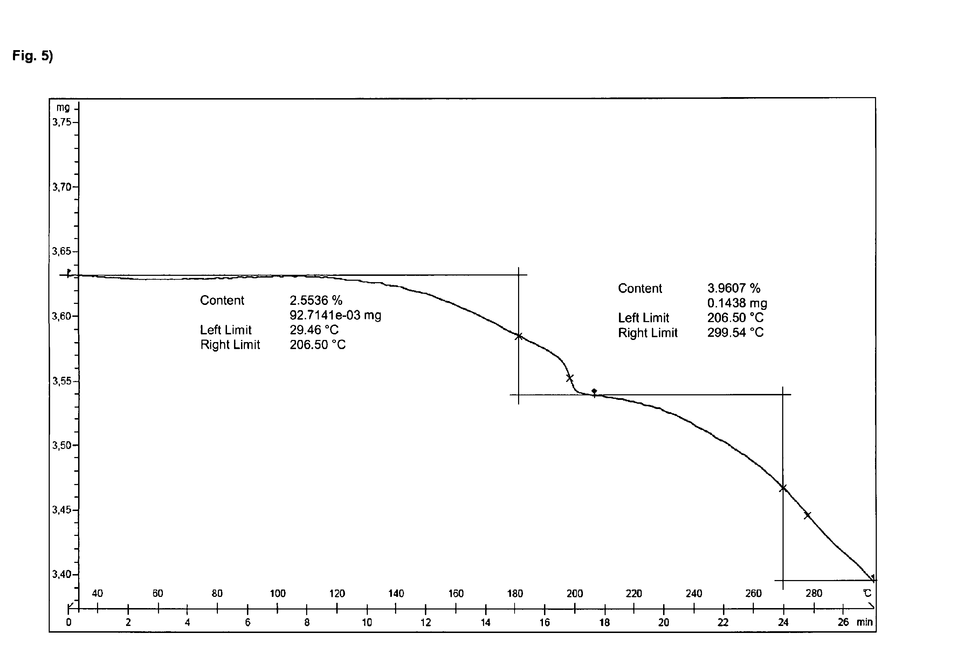

The TG analysis of the crystalline form according to the invention shows weight loss at temperatures lower than the melting point according to the ethanol desolvatation (see figure 5).

### XRPD

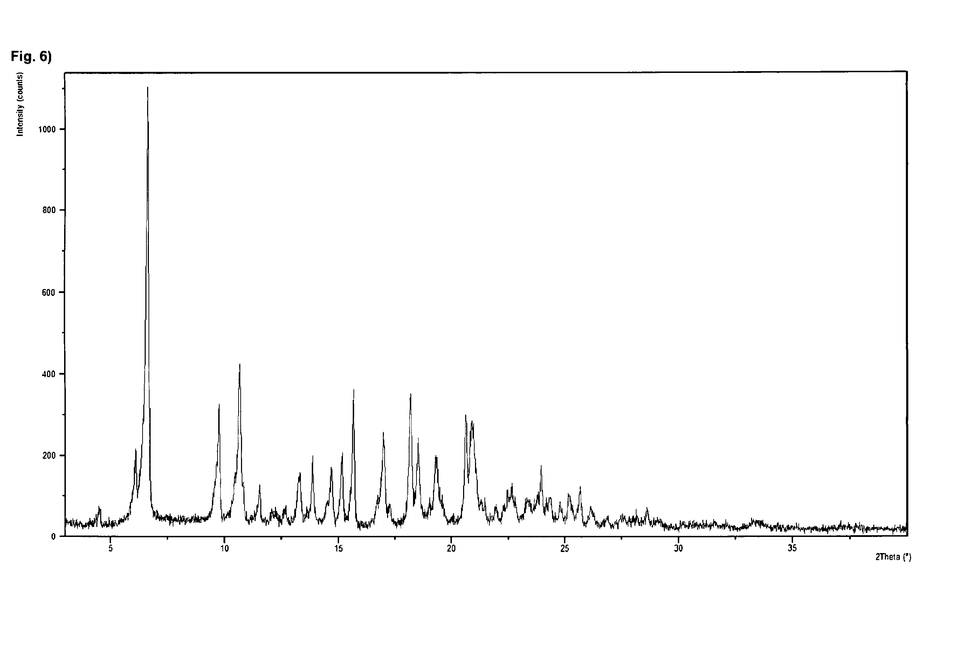

XRPD analysis was performed using a Philips X'Pert diffractometer with Cu K_{α} radiation in Bragg-Brentano geometry. The system is equipped with a monodimensional, real time multiple strip detector. The diffractogram was recorded from 3° to 40°(2θ) at a scan rate of 17.6° per minute (see figure 6).

List of selected peaks (only peaks with relative intensity greater than or equal to 1 % are indicated):

| **2θ (°)** | **d (Å)** | **I (%)** | | **2θ (°)** | **d (Å)** | **I (%)** |
|---|---|---|---|---|---|---|
| 4.51 | 19.58 | 4 | | 19.29 | 4.60 | 27 |
| 6.09 | 14.51 | 15 | | 20.60 | 4.31 | 36 |
| 6.64 | 13.29 | 100 | | 20.85 | 4.25 | 33 |
| 9.75 | 9.06 | 31 | | 21.95 | 4.04 | 4 |
| 10.67 | 8.28 | 47 | | 22.64 | 3.92 | 16 |
| 11.53 | 7.67 | 11 | | 23.35 | 3.80 | 5 |
| 12.05 | 7.34 | 3 | | 23.95 | 3.71 | 16 |
| 12.66 | 6.98 | 4 | | 24.35 | 3.65 | 9 |
| 13.26 | 6.67 | 16 | | 24.77 | 3.59 | 4 |
| 13.87 | 6.38 | 19 | | 25.15 | 3.53 | 8 |
| 14.68 | 6.03 | 17 | | 25.65 | 3.47 | 15 |
| 15.16 | 5.84 | 25 | | 26.12 | 3.41 | 7 |
| 15.66 | 5.65 | 42 | | 26.82 | 3.32 | 1 |
| 17.03 | 5.20 | 25 | | 27.50 | 3.24 | 3 |
| 17.24 | 5.14 | 7 | | 28.60 | 3.12 | 4 |
| 18.15 | 4.88 | 38 | | 31.55 | 2.83 | 2 |
| 18.49 | 4.79 | 32 | | 33.23 | 2.69 | 2 |

### Example 3: Telmisartan - Atenolol - H₂O (1:1:1) (salt)

### Example 3.1: Precipitation in H₂O of atenolol hydrochloride and sodium salt of telmisartan

To an assay tube equipped with magnetic stirrer containing atenolol hydrochloride (12 mg, 0.04 mmol) and sodium salt of telmisartan (20 mg, 0.04 mmol, 1.0 eq), was added deionized water (0.5 mL). The resultant solution started to crystallize rapidly and the mixture was stirred at room temperature for 15 hours. The solid was filtered with a sintered funnel (porosity 4), washed with water (0.2 mL) and dried under vacuum at room temperature to give salt of telmisartan - atenolol - H₂O (1:1:1) as a white solid (18 mg, 56% yield).

### Example 3.2: Precipitation in H₂O of atenolol hydrochloride and sodium salt of telmisartan (1.8 g scale)

To a round-bottom flask equipped with magnetic stirrer containing atenolol hydrochloride (677 mg, 2.23 mmols) and sodium salt of telmisartan (1.2 g, 2.23 mmols, 1.0 eq), was added deionized water (15 mL). The resultant solution was seeded with form obtained in the Example 3.1 before starting to crystallize and the mixture was stirred at room temperature for 8 hours. The solid was filtered with a sintered funnel (porosity 4), washed with ethanol (3 x 2 mL) and dried under vacuum at 45°C during 15 hours to give form salt of telmisartan - atenolol - H₂O (1:1:1) as a white solid (1.34 g, 75% yield).

### Slurrying in ACN

To an assay tube equipped with magnetic stirrer containing atenolol (20 mg, 0.07 mmol, 1.2 eq) and telmisartan (32 mg, 0.06 mmol), was added acetonitrile (0.5 mL). The resultant slurring was seeded with form obtained in the Example 3.1 and stirred at room temperature for 6 hours. The solid was filtered with a sintered funnel (porosity 4), washed with acetonitrile (0.2 mL) and dried under vacuum at room temperature to give salt of atenolol - telmisartan - H₂O (1:1:1) as a white solid (39 mg, 79% yield).

### ¹H NMR

Proton nuclear magnetic resonance analyses were recorded in deuterated dimethyl sulphoxide (DMSO) in a Varian Mercury 400 spectrometer, equipped with a broadband probe ATB 1H/19F/X of 5 mm. Spectra were acquired dissolving 5-10 mg of sample in 0.6 mL of deuterated solvent.

¹H NMR (DMSO, 400 MHz): δ = 7.73 (s, 1H); 7.66 (d, *J* = 7.4 Hz, 1H); 7.58 (d, *J* = 7.2 Hz, 1 H); 7.60-7.51 (m, 2H); 7.47 (s, 1 H); 7.44-7.31 (m, 5H); 7.29-7.19 (m, 3H); 7.18-7.12 (m, 4H); 6.67-6.82 (m, 2H); 6.80 (s, br, 1 H); 5.60 (s, 2H); 3.98-3.83 (m, 3H); 3.82 (s, 3H); 3.28 (s, 3H); 2.96-2.67 (m, 5H); 2.63 (s, 3H); 1.83 (sx, *J* = 7.2 Hz, 2H), 1.04-0.97 (m, 9H).

### IR

FTIR spectra were recorded using a Thermo Nicolet Nexus 870 FT-IR, equipped with a beamsplitter KBr system, a 35mW He-Ne laser as the excitation source and a DTGS KBr detector. The spectra were acquired in 32 scans at a resolution of 4 cm⁻¹.

IR (KBr): v = 3376.3 (vs), 3159.5 (s), 3054.9 (s), 2967.7 (s), 2866.2 (2), 1670.0 (m), 1566.9 (s), 1509.0 (m), 1459.3 (m), 1385.8 (w), 1331.6 (s), 1231.8 (m), 1177.2 (vs), 770.9 (vs), 756.1 (s) cm⁻¹.

### DSC

DSC analyses were recorded with a Mettler DSC822^{e}. A sample of 2.1950 mg was weighed into 40 µL aluminium crucible with a pinhole lid and was heated, under nitrogen (50 mL/min), at 10°C/min from 30 to 300 °C.

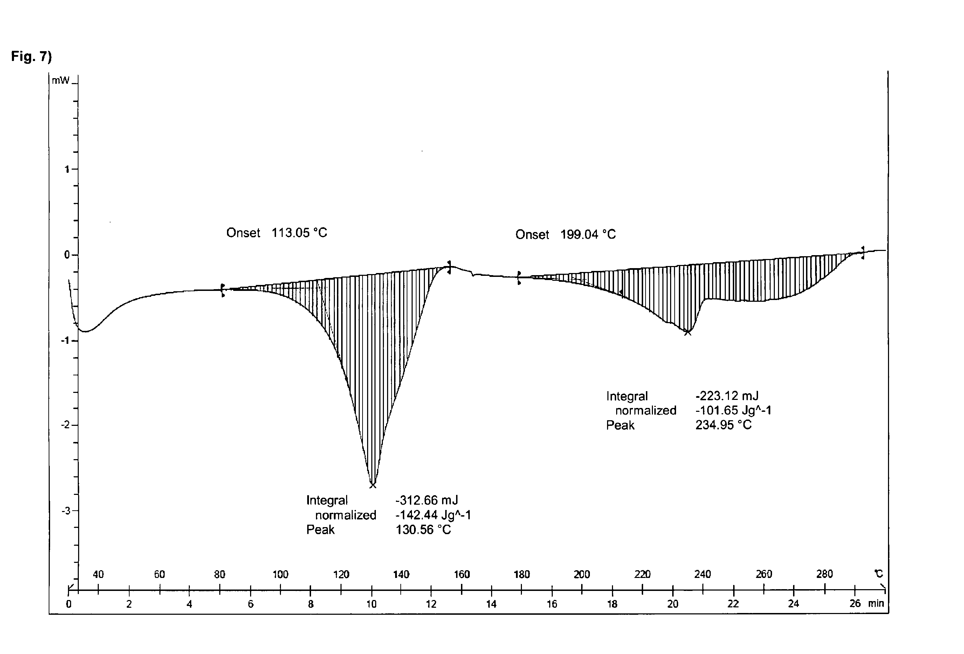

The novel type of crystal of the present invention is characterized in that the endothermic sharp peak corresponding to the melting point + dehydration has an onset at 113.05 °C (fusion enthalpy -142.44 J/g), measured by DSC analysis (10 °C/min) (see figure 7).

### TG

Thermogravimetric analyses were recorded in a thermogravimetric analyzer Mettler TGA/SDTA851^{e}. A sample of 3.9957 mg was weighed into a 70 µL alumina crucible with a pinhole lid and was heated at 10°C/min from 30 to 250°C, under nitrogen (50 mL/min).

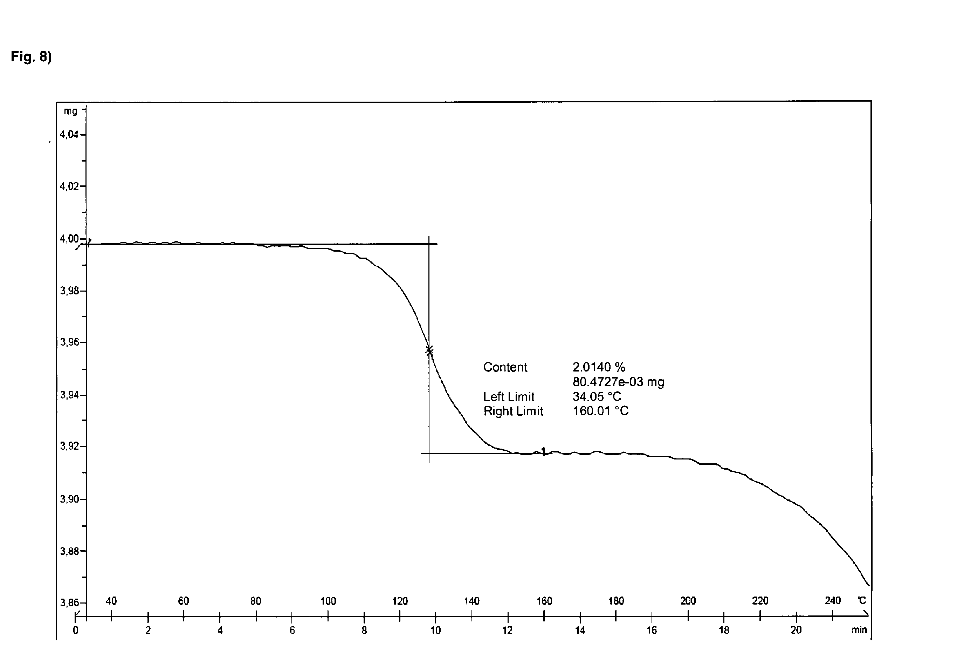

The TG analysis of the crystalline form according to the invention shows weight loss at temperatures higher than the melting point according to the dehydration (see figure 8).

### XRPD

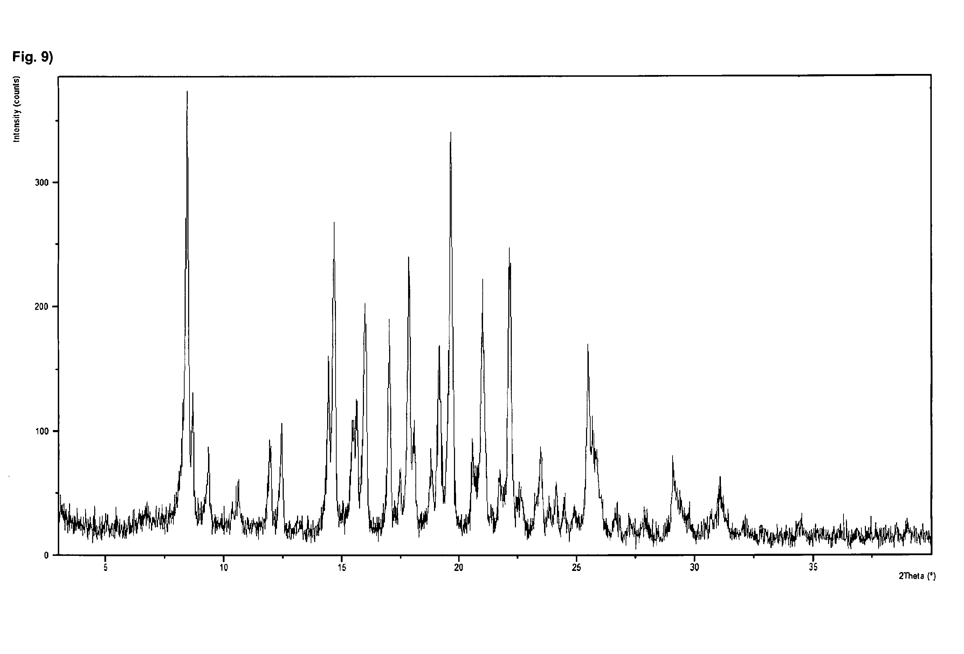

XRPD analysis was performed using a Philips X'Pert diffractometer with Cu K_{α} radiation in Bragg-Brentano geometry. The system is equipped with a monodimensional, real time multiple strip detector. The diffractogram was recorded from 3° to 40° at a scan rate of 17.6° per minute (see figure 9).

List of selected peaks (only peaks with relative intensity greater than or equal to 1 % are indicated):

| **2θ (°)** | **d (Å)** | **I (%)** | **2θ (°)** | **d (Å)** | **I (%)** |
|---|---|---|---|---|---|
| 8.45 | 10.46 | 100 | 22.19 | 4.01 | 59 |
| 8.68 | 10.18 | 28 | 22.58 | 3.94 | 7 |
| 9.34 | 9.47 | 17 | 23.48 | 3.79 | 19 |
| 10.35 | 8.55 | 10 | 23.80 | 3.74 | 4 |
| 10.60 | 8.35 | 9 | 24.11 | 3.69 | 12 |
| 11.95 | 7.41 | 20 | 24.47 | 3.64 | 7 |
| 12.44 | 7.11 | 26 | 24.90 | 3.58 | 5 |
| 14.43 | 6.14 | 43 | 25.48 | 3.50 | 45 |
| 14.67 | 6.04 | 69 | 25.66 | 3.47 | 37 |
| 15.48 | 5.72 | 26 | 25.82 | 3.45 | 28 |
| 15.62 | 5.67 | 29 | 26.64 | 3.35 | 5 |
| 15.99 | 5.54 | 56 | 27.26 | 3.27 | 5 |
| 17.02 | 5.21 | 49 | 27.89 | 3.20 | 5 |
| 17.47 | 5.08 | 13 | 28.43 | 3.14 | 1 |
| 17.86 | 4.97 | 62 | 29.10 | 3.07 | 17 |
| 18.07 | 4.91 | 22 | 29.76 | 3.00 | 12 |
| 18.81 | 4.72 | 16 | 30.69 | 2.91 | 5 |
| 19.14 | 4.64 | 45 | 31.09 | 2.88 | 12 |
| 19.66 | 4.52 | 98 | 32.16 | 2.78 | 2 |
| 20.56 | 4.32 | 19 | 34.43 | 2.61 | 4 |
| 20.99 | 4.23 | 53 | 38.49 | 2.34 | 5 |
| 21.71 | 4.09 | 13 | 38.97 | 2.31 | 4 |

### Single crystal X-ray diffraction

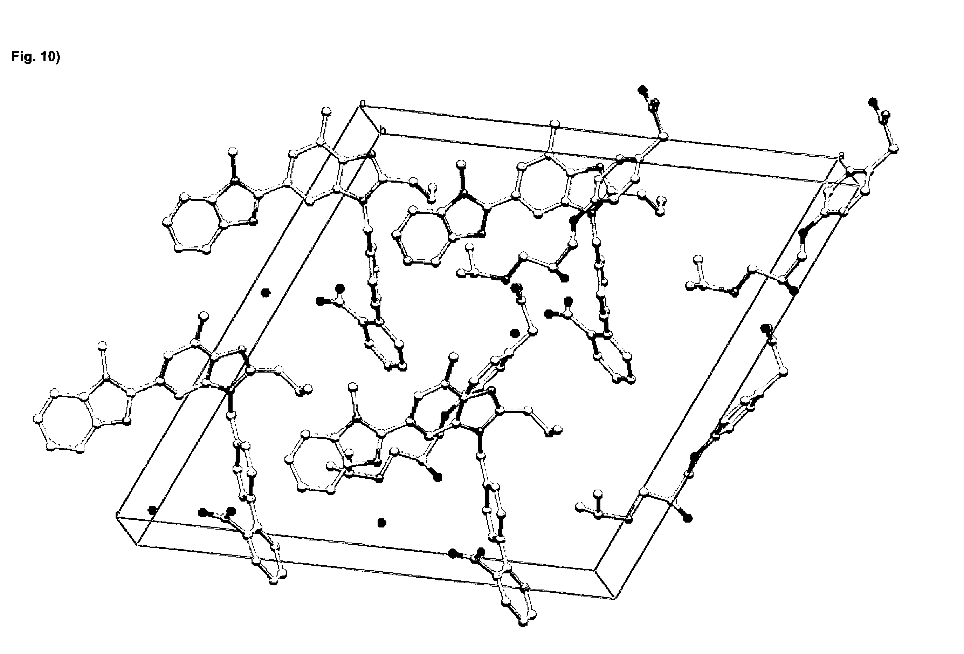

The crystal structure of telmisartan - atenolol - H₂O (1:1:1) salt has been determined from single crystal X-ray diffraction data. The colourless prism used (0.25 x 0.19 x 0.17 mm) was obtained from the evaporation of a solution in ethanol of atenolol and telmisartan (see figure 10).

| Crystal system | Monoclinic |
|---|---|
| Space group | *Cc* |
| a (Å) | 22.7566(17) |
| b (Å) | 9.2383(7) |
| c (Å) | 22.1794(17) |
| β (°) | 113.980(2) |
| Volume (Å³) | 4260.4(9) |
| Z | 4 |
| D calc. (Mg/m³) | 1.25 |
| N. of refl. | 14567 |
| Refl. with I > 2σ(I) | 5171 |
| R (I > 2σ(I)) | 5.34 |

Analysis was performed at room temperature using a Bruker Smart Apex diffractometer with graphite monochromated Mo K_{α} radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0).

The structure was solved with direct methods and least-squares refinement of Fₒ² against all measured intensities was carried out (program used: SHELXTL-NT 6.1). All non-hydrogen atoms were refined with anisotropic displacement parameters. The isopropyl group of the atenolol molecule was found to be disordered.

### Relevant structural data:

The unit cell contents of the crystal structure of this form are depicted in figure 10 (hydrogen atoms have been omitted for clarity; program used: *Mercury* 2.4, C.F. Macrae, I.J. Bruno, J.A. Chisholm, P.R. Edgington, P. McCabe, E. Pidcock, L. Rodriguez-Monge, R. Taylor, J. van de Streek and P.A. Wood, J. Appl. Cryst., 41, 2008, 466-470).

### Example 4: Telmisartan - Bisoprolol - Fumaric Acid (2:1:1) (co-crystal)

Telmisartan used in the following experiments was polymorph A (US 2006/0276525 A1).

### Example 4.1: Slurrying in CHCl₃ of bisoprolol hemifumarate and telmisartan (A):

To an assay tube equipped with magnetic stirrer containing telmisartan (A) (30 mg, 0.06 mmol, 2 eq)) and bisoprolol hemifumarate (22 mg, 0.03 mmol, 1 eq) was added chloroform (0.5 mL). The resultant slurrying was stirred at room temperature for 15 hours The solid was centrifuged and dried under vacuum at room temperature to give co-crystal of telmisartan-bisoprolol - fumaric acid (2:1:1) as a white solid (32 mg, 75% yield).

### Example 4.2: Slurrying in CHCl₃ of bisoprolol hemifumarate and telmisartan (A):

To an assay tube equipped with magnetic stirrer containing a suspension of telmisartan (A) (106 mg, 0.20 mmol, 0.7 eq) in chloroform (0.85 mL) was added a solution of bisoprolol hemifumarate (225 mg, 0.30 mmol, 1 eq) in chloroform (0.85 mL). The resultant slurrying was seeded with form obtained in Example 4.1 and stirred at 35°C for 3 hours and 30 minutes with sonication period of 5 minutes every hour. Then, the solid was filtered with a sintered funnel (porosity 4) and dried under atmospheric pressure at room temperature 15 hours to give co-crystal of telmisartan- bisoprolol - fumaric acid (2:1:1) as a white solid (62 mg, 42% yield).

### Example 4.3: Slurrying in CHCl₃ of bisoprolol hemifumarate and telmisartan (A (3.4 g scale):

To a round-bottom flask equipped with magnetic stirrer containing a suspension of telmisartan (A) (1.1 g, 2.14 mmol, 0.7 eq) in chloroform (8.6 mL) was added a solution of bisoprolol hemifumarate (2.3 g, 3.00 mmol, 1 eq) in chloroform (8.6 mL). The resultant slurrying was seeded with form obtained in Example 4.1 and stirred at 35°C for 3 hours and 30 minutes with sonication period of 5 minutes every hour. Then, the solid was filtered with a sintered funnel (porosity 4) and dried under vacuum of 500 mbar 2 hours and atmospheric pressure at room temperature to give form #274 bisoprolol - fumaric acid - telmisartan (1:1:2) as a white solid (834 mg, 53% yield).

### ¹H NMR

Proton nuclear magnetic resonance analyses were recorded in deuterated dimethyl sulphoxide (DMSO) in a Varian Mercury 400 spectrometer, equipped with a broadband probe ATB 1H/19F/X of 5 mm. Spectra were acquired dissolving 5-10 mg of sample in 0.6 mL of deuterated solvent.

¹H NMR (DMSO, 400 MHz): δ = 7.72 (s, 1H); 7.67 (d, *J* = 7.6 Hz, 2H); 7.58 (d, *J* = 7.6 Hz, 1H); 7.51 (dt, *J* = 7.6 Hz, *J* = 1.2 Hz, 1H); 7.47 (s, 2H); 7.41 (dt, *J* = 7.6 Hz, *J* = 1.2 Hz, 1 H); 7.35-7.28 (m, 3H); 7.28-7.20 (m, 3H); 7.17 (d, *J* = 7.6 Hz, 2H); 6.92 (d, *J* = 8.8 Hz, 1H); 6.55 (s, 1H); 5.62 (s, 2H); 4.40 (s, 1H); 4.20-4.10 (m, 1H); 4.01-3.90 (m, 1H); 3.82 (s, 3H); 3.54 (sept., *J* = 6.4 Hz, 1 H); 3.48 (s, 2H); 3.25 (quint., *J* = 6.4 Hz, 0.5H); 3.07 (dd, *J* = 12.4 Hz, *J* = 2.8 Hz, 1 H); 2.93 (t, *J* = 7.6 Hz, 2H); 2.63 (s, 3H); 1.82 (sx, *J* = 7.6 Hz, 2H); 1.25-1.15 (m, 3H); 1.07 (d, *J* = 5.6 Hz, 3H); 1.00 (t, *J* = 7.6 Hz, 3H).

### IR

FTIR spectra were recorded using a Thermo Nicolet Nexus 870 FT-IR, equipped with a beamsplitter KBr system, a 35mW He-Ne laser as the excitation source and a DTGS KBr detector. The spectra were acquired in 32 scans at a resolution of 4 cm⁻¹.

IR (KBr): v = 3411.3 (br), 3059.6 (s), 2968.7 (w), 2929.4 (m), 2870.2 (w), 2466.7 (br), 2348.8 (vs), 1693.1 (w), 1464.9 (w), 1246.2 (w), 1041.1 (s), 913.6 (s), 742.4 (w), 724.4 (s), 704.7 (s) cm⁻¹.

### DSC

DSC analyses were recorded with a Mettler DSC822^{e}. A sample of 2.7720 mg was weighed into 40 µL aluminium crucible with a pinhole lid and was heated, under nitrogen (50 mL/min), at 10°C/min from 30 to 300 °C.

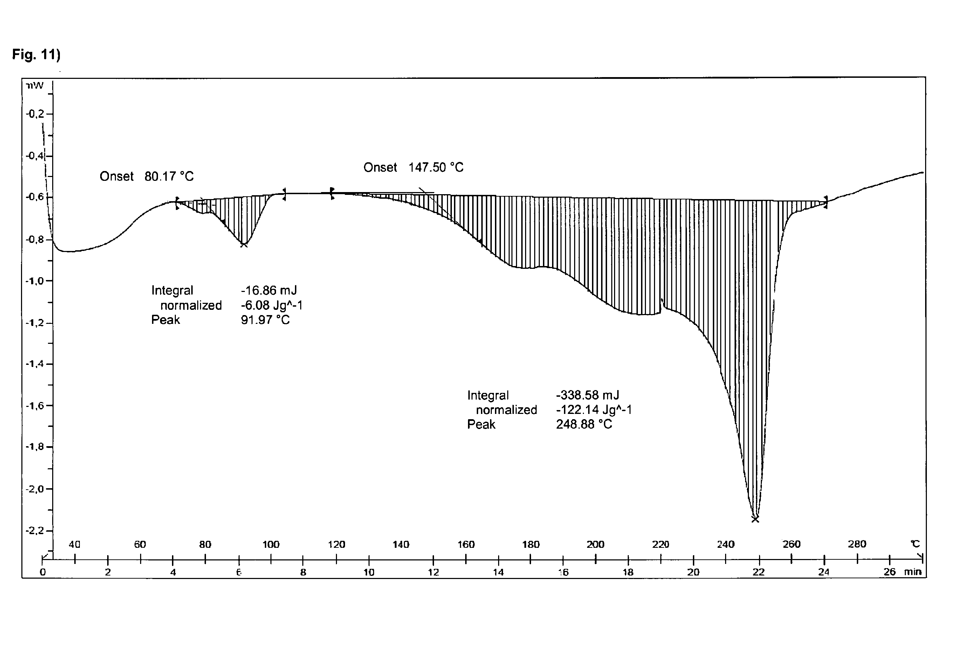

The novel type of crystal of the present invention is characterized in that the endothermic peak (onset at 80.17°C - enthalpy -6.08 J/g) measured by DSC analysis (10 °C/min) (see figure 11). This peak could correspond to the co-crystal transformation in the crystalline form of telmisartan and amorphous salt between bisoprolol and fumaric acid. Then, the endothermic peak with an onset at 147.5 °C (enthalpy -122.14 J/g) measured by DSC analysis (10 °C/min) (see figure 11) corresponds to chemical degradation, that finally leads to the melting of telmisartan.

### TG

Thermogravimetric analyses were recorded in a thermogravimetric analyzer Mettler TGA/SDTA851^{e}. A sample of 4.9161 mg was weighed into a 70 µL alumina crucible with a pinhole lid and was heated at 10°C/min from 30 to 300°C, under nitrogen (50 mL/min).

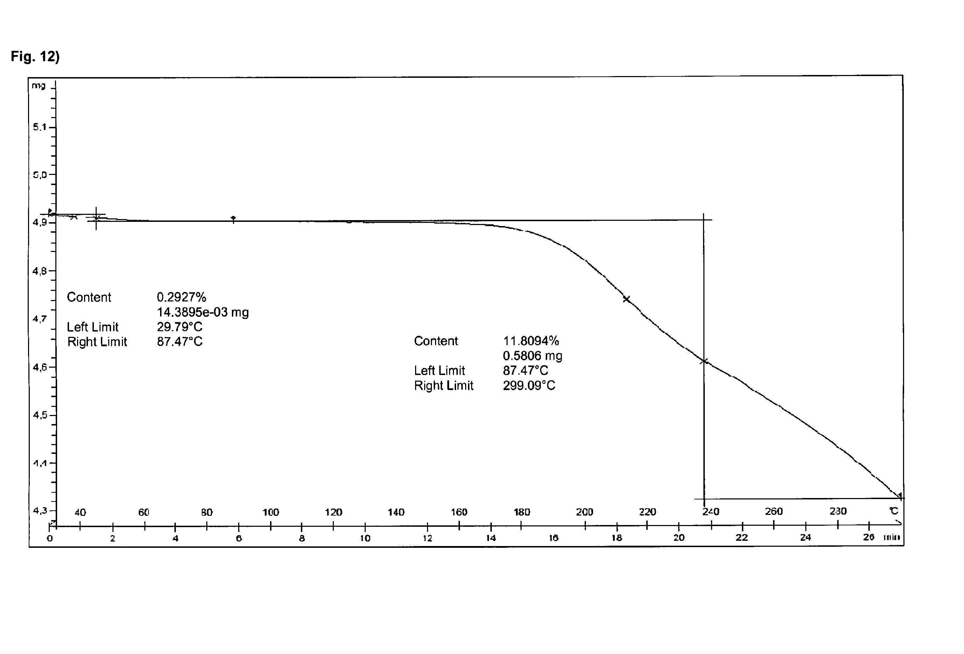

The TG analysis of the crystalline form according to the invention shows weight loss of 0.293% before the chemical degradation (see figure 12).

### XRPD

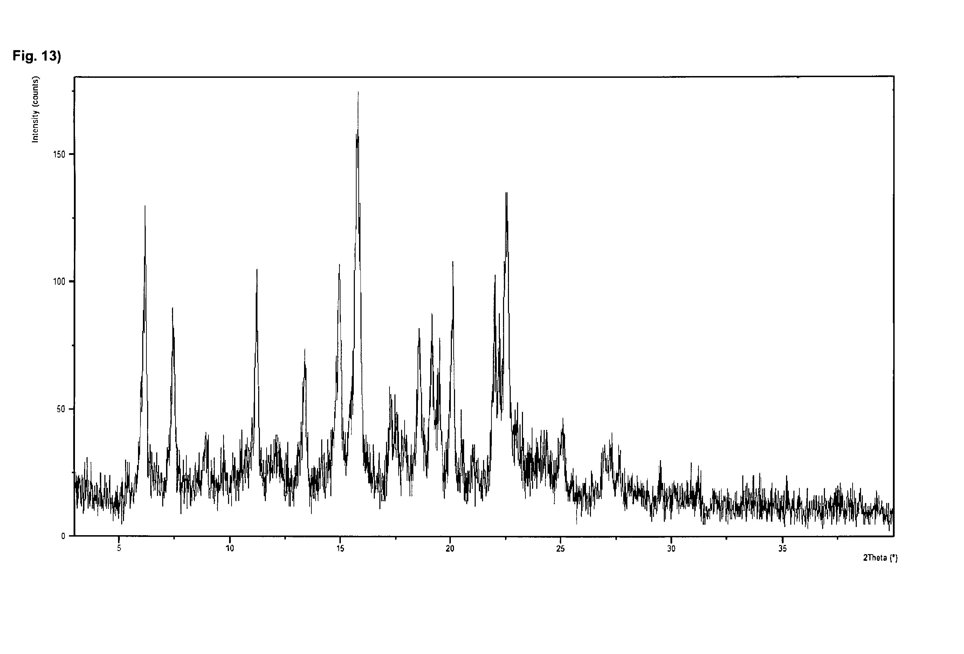

XRPD analysis was performed using a Philips X'Pert diffractometer with Cu Kₐ radiation in Bragg-Brentano geometry. The system is equipped with a monodimensional, real time multiple strip detector. The diffractogram was recorded from 3° to 40° at a scan rate of 17.6° per minute (see figure 13).

List of selected peaks (only peaks with relative intensity greater than or equal to 1 % are indicated):

| **2θ (°)** | **d (Å)** | **I (%)** |
|---|---|---|
| 6,17 | 14,34 | 74 |
| 7,45 | 11,86 | 45 |
| 8,93 | 9,91 | 13 |
| 11,21 | 7,89 | 51 |
| 13,39 | 6,61 | 36 |
| 14,94 | 5,93 | 62 |
| 15,75 | 5,63 | 100 |
| 17,24 | 5,14 | 23 |
| 18,56 | 4,78 | 40 |
| 19,12 | 4,64 | 36 |
| 20,10 | 4,42 | 51 |
| 21,97 | 4,05 | 50 |
| 22,55 | 3,94 | 74 |
| 25,08 | 3,55 | 18 |
| 27,09 | 3,29 | 11 |

### Example 5: Telmisartan - Bisoprolol - Fumaric Acid (4:1:2) (co-crystal)

### Example 5.1: Slurrying in CHCl₃ of bisoprolol hemifumarate and telmisartan (A):

To an assay tube equipped with magnetic stirrer containing telmisartan (A) (50.0 mg, 0.1 mmol, 1 eq)) and bisoprolol hemifumarate (75.0 mg, 0.1 mmol, 1 eq) was added chloroform (0.62 mL). The resultant slurrying was seeded with form obtained in Example 4.1 and stirred at room temperature for 15 hours The solid was filtered with a sintered funnel (porosity 4) and dried under vacuum at room temperature to give co-crystal telmisartan - bisoprolol - fumaric acid (2:1:1) as a white solid (42 mg, 57% yield).

After that, the solid was washed with toluene (2 x 5 volumes) and dried under vacuum at room temperature to give co-crystal of telmisartan - bisoprolol - fumaric acid (4:2:1) as a white solid (31 mg, 47% yield).

### Example 5.2: Slurrying in CHCl₃ of bisoprolol hemifumarate and telmisartan(A) (3.4 g scale):

To a round-bottom flask equipped with magnetic stirrer containing a suspension of telmisartan (A) (1.1 g, 2.14 mmol, 0.7 eq) in 2.5 volumes of chloroform (8.6 mL) was added a solution of bisoprolol hemifumarate (2.3 g, 3.00 mmol, 1 eq) in 2.5 volumes of chloroform (8.6 mL). The resultant slurrying was seeded with form obtained in Example 5.1 and stirred at 35°C for 3 hours with sonication period of 5 minutes every hour. After that, one volume of chloroform (3.5 mL) was added to the mixture and stirred 10 more minutes. The solid was filtered with a sintered funnel (porosity 4) and dried under vacuum of 650 mbar 2 hours and atmospheric pressure at room temperature to give form bisoprolol - fumaric acid - telmisartan (0.8:1:2) as a white solid. The solid was washed with toluene (9 x 5 volumes) and dried under vacuum at room temperature and at 50°C until to give co-crystal of telmisartan - bisoprolol - fumaric acid (4:2:1) as a white solid (600 mg, 43% yield).

### ¹H NMR

Proton nuclear magnetic resonance analyses were recorded in deuterated dimethyl sulphoxide (DMSO) in a Varian Mercury 400 spectrometer, equipped with a broadband probe ATB 1H/19F/X of 5 mm. Spectra were acquired dissolving 5-10 mg of sample in 0.6 mL of deuterated solvent.

¹H NMR (DMSO, 400 MHz): δ = 7.72 (s, 1H); 7.68 (t, *J* = 7.6 Hz, 2H); 7.58 (d, *J* = 7.6 Hz, 1 H); 7.52 (t, *J* = 7.6 Hz, 1H); 7.48(s, 1H); 7.42 (t, *J* = 7.6 Hz, 1 H); 7.36-7.20 (m, 5.5H); 7.18 (d, *J* = 7.6 Hz, 2H); 6.92 (d, *J* = 8.4 Hz, 0.5H); 6.57 (s, 1H); 5.62 (s, 2H); 4.40 (s, 0.5H); 4.17-4.07 (m, 0.5H); 4.01-3.91 (m, 0.5H); 3.82 (s, 3H); 3.54 (sept., *J* = 6.4 Hz, 0.5H); 3.48 (s, 1H); 3.25 (quint, *J* = 6.4 Hz, 0.25H); 3.12-3.04 (m, 0.5H); 2.93 (t, *J* = 7.6 Hz, 2H); 2.63 (s, 3H); 1.82 (sx, *J* = 7.6 Hz, 2H); 1.26-1.15 (dd, *J* = 6.4 Hz, *J* = 4.0 Hz, 1.5H); 1.07 (d, *J* = 5.6 Hz, 1.5H); 1.00 (t, *J* = 7.6 Hz, 3H).

### IR

FTIR spectra were recorded using a Thermo Nicolet Nexus 870 FT-IR, equipped with a beamsplitter KBr system, a 35mW He-Ne laser as the excitation source and a DTGS KBr detector. The spectra were acquired in 32 scans at a resolution of 4 cm⁻¹.

IR (KBr): v = 3059.9 (vs), 2966.6 (s), 2929.3 (s), 2870.5 (s), 2459.9 (br), 1700.8 (m), 1599.6 (s), 1560.4 (s), 1465.2 (m), 1447.9 (m), 1297.8 (m), 1246.3 (w), 1129.2 (w), 1086.5 (m), 761.0 (w), 704.7 (s) cm⁻¹.

### DSC

DSC analyses were recorded with a Mettler DSC822^{e}. A sample of 2.3340 mg was weighed into 40 µL aluminium crucible with a pinhole lid and was heated, under nitrogen (50 mL/min), at 10°C/min from 30 to 300 °C.

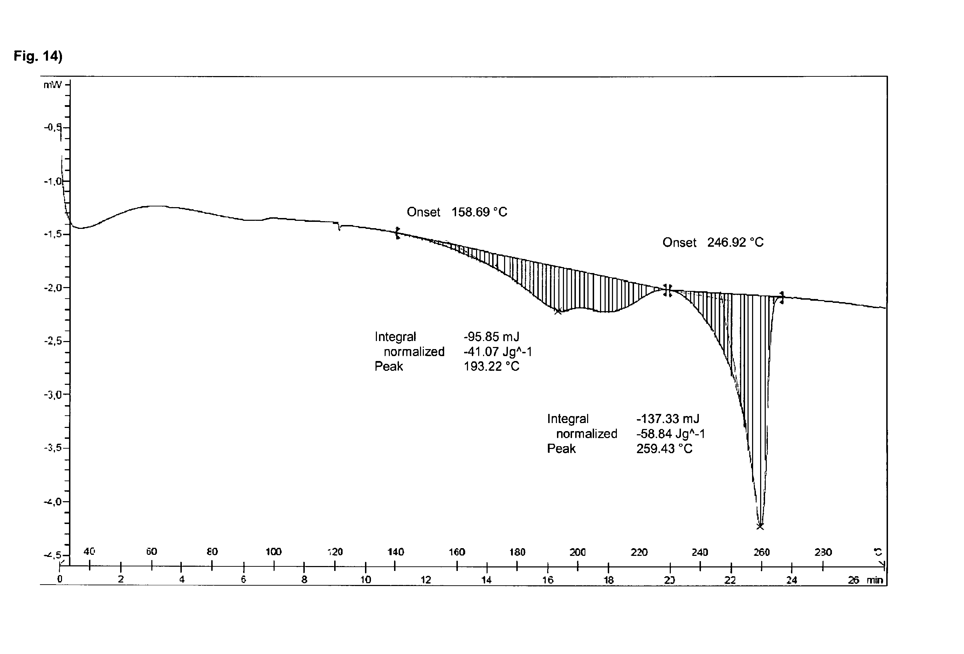

The novel type of crystal of the present invention is characterized by an endothermic peak corresponding to a chemical degradation with an onset at 158.69 °C (enthalpy -41.07 J/g) measured by DSC analysis (10 °C/min). (see figure 14). Then, another endothermic peak with an onset at 246.92°C (enthalpy -58.84 J/g) was observed corresponding to the melting of recrystallized telmisartan (see figure 14).

### TG

Thermogravimetric analyses were recorded in a thermogravimetric analyzer Mettler TGA/SDTA851^{e}. A sample of 2.1725 mg was weighed into a 70 µL alumina crucible with a pinhole lid and was heated at 10°C/min from 30 to 300°C, under nitrogen (50 mL/min).

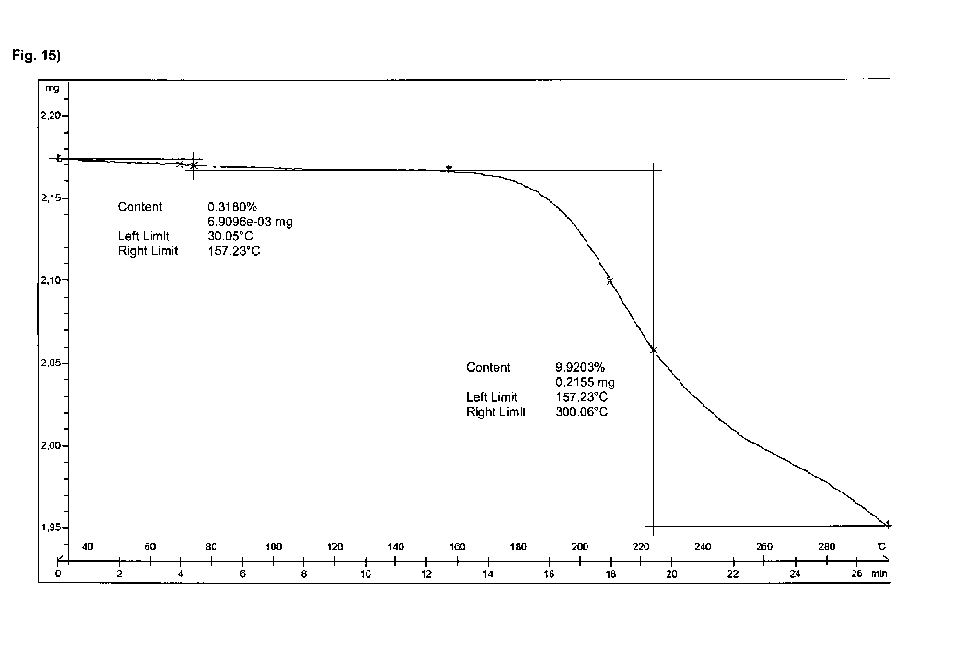

The TG analysis of the crystalline form according to the invention shows a progressive weight loss of 0.318% before the chemical degradation (0.5 eq, H₂O (see figure 15).

### XRPD

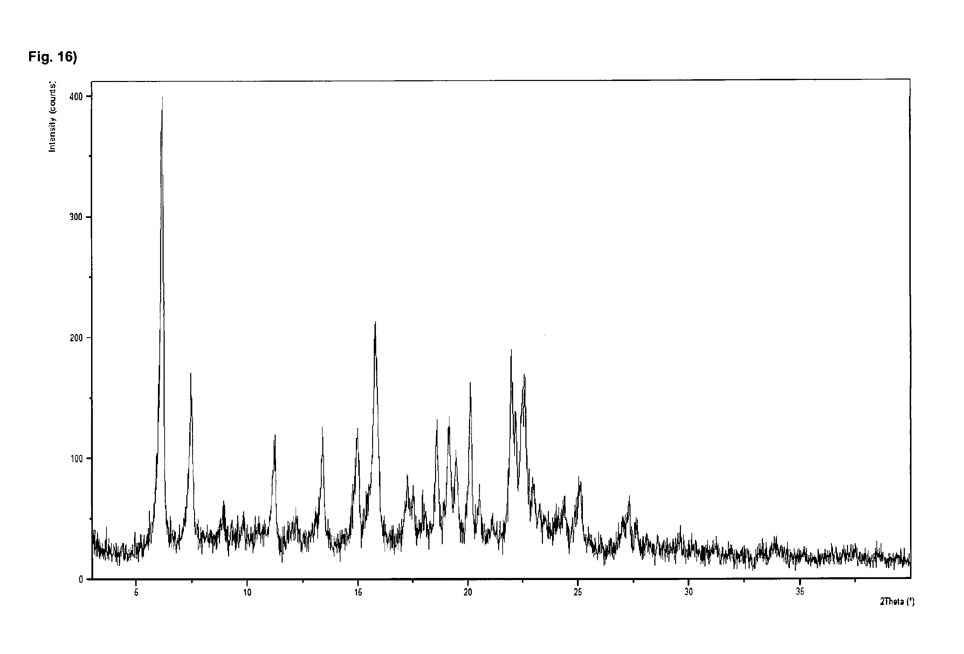

XRPD analysis was performed using a Philips X'Pert diffractometer with Cu K_{α} radiation in Bragg-Brentano geometry. The system is equipped with a monodimensional, real time multiple strip detector. The diffractogram was recorded from 3° to 40° at a scan rate of 17.6° per minute (see figure 16).

List of selected peaks (only peaks with relative intensity greater than or equal to 1 % are indicated):

| **2θ (°)** | **d (Å)** | **I (%)** |
|---|---|---|
| 6,18 | 14,30 | 100 |
| 7,47 | 11,84 | 34 |
| 8,85 | 9,99 | 6 |
| 11,24 | 7,87 | 22 |
| 12,13 | 7,29 | 3 |
| 13,39 | 6,61 | 26 |
| 14, 98 | 5,91 | 26 |
| 15,79 | 5,61 | 51 |
| 17,22 | 5,15 | 12 |
| 18,59 | 4,77 | 24 |
| 19,15 | 4,63 | 27 |
| 19,48 | 4,56 | 19 |
| 20,10 | 4,42 | 35 |
| 20,49 | 4,33 | 9 |
| 21,96 | 4,05 | 44 |
| 22,51 | 3,95 | 32 |
| 24,37 | 3,65 | 7 |
| 25,10 | 3,55 | 14 |
| 27,27 | 3,27 | 12 |
| 27,62 | 3,24 | 6 |

### Example 6: Candensartan cilexetil - Nebivolol (1:1) (salt)

### Example 6.1: Crystallization in isopropanol

To an assay tube equipped with magnetic stirrer containing nebivolol free base (20 mg, 0.05 mmol) in solution in isopropanol (0.8 mL) at 80°C was added candesartan cilexetil (30 mg, 0.05 mmol). The resultant mixture was stirred until a complete solution was achieved. Then, it was cooled to room temperature and the suspension obtained was stirred for 16 hours. The precipitated solid was filtered with a sintered funnel (porosity 4), washed twice with isopropanol and dried under vacuum at room temperature to give a mixture of candesartan cilexetil - nebivolol - isopropanol solvate (1:1:1) and candensartan cilexetil - nebivolol (1:1) (salt) as a white solid (38 mg, 75% yield).

### Example 6.2: Crystallization in ethanol

To a round-bottom flask equipped with magnetic stirrer containing nebivolol free base (41 mg, 0.10 mmol) and candesartan cilexetil (61 mg, 0.10 mmol) was added ethanol (1 mL) before heating at 45°C. The resulting solution was seeded with the mixture obtained in the Example 1 (mixture of candesartan cilexetil - nebivolol - isopropanol solvate (1:1:1) and candensartan cilexetil - nebivolol (1:1) (salt)), cooled to room temperature and then stirred for 5 hours. The precipitated solid was filtered with a sintered funnel (porosity 4), washed twice with ethanol furnishing an unstable solvate of EtOH. Then a drying under vacuum at room temperature gave candensartan cilexetil - nebivolol (1:1) (salt) as a white solid (74 mg, 73% yield).

### Example 6.3: Crystallization in ethanol

To a round-bottom flask equipped with magnetic stirrer containing nebivolol free base (500 mg, 1.23 mmol) and candesartan cilexetil (754 mg, 1.23 mmol) was added ethanol (12.5 mL) before heating at 45°C. The resulting solution was seeded with the crystalline obtained in Example 2, cooled to room temperature and then stirred for 5 hours. The precipitated solid was filtered with a sintered funnel (porosity 3) furnishing an unstable solvate of EtOH. Then a drying under vacuum at 60°C gave candensartan cilexetil - nebivolol (1:1) (salt) as a white solid (1.02 g, 81% yield).

### ¹H NMR

Proton nuclear magnetic resonance analyses were recorded in deuterated dimethyl sulfoxide (DMSO) in a Varian Mercury 400 spectrometer, equipped with a broadband probe ATB 1H/19F/X of 5 mm. Spectra were acquired dissolving 5-10 mg of sample in 0.6 mL of deuterated solvent.

¹H NMR (DMSO, 400 MHz): δ = 7.73 (d, *J* = 7.8 Hz, 1H); 7.58-7.50 (m, 1H); 7.44 (d, *J* = 7.8 Hz, 1H); 7.41-7.33 (m, 2H); 7.29-7.23 (m, 1H); 7.20 (t, *J* = 7.8, 1H); 7.00 (d, *J* = 9.2 Hz, 2H); 6.97-6.85 (m, 4H); 6.84-6.70 (m, 5H); 5.48 (s, 2H); 4.67-4.52 (m, 3H); 4.03-3.92 (m, 2H); 3.92-3.83 (m, 2H); 3.23-3.14 (m, 1H); 3.13-3.02 (m, 2H); 3.01-2.91 (m, 1H); 2.87-2.69 (m, 4H); 2.16-2.04 (m, 1H); 1.98-1.88 (m, 1H); 1.88-1.5 (m, 6H); 1.51-1.15 (m, 12H).

### IR

FTIR spectrum was recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

The sample (KBr pellets) shows a Fourier Transform Infra Red spectrum with absorption bands at 3390 (m), 3059 (br), 2938 (m), 2861 (m), 1762 (s), 1549 (s), 1492 (s), 1215 (s), 1084 (m) cm⁻¹.

### DSC

DSC analyses were recorded with a Mettler DSC822^{e}. A sample of 2.8160 mg was weighed into 40 µL aluminium crucible with a pinhole lid and was heated, under nitrogen (50 mL/min), at 10°C/min from 30 to 300 °C.

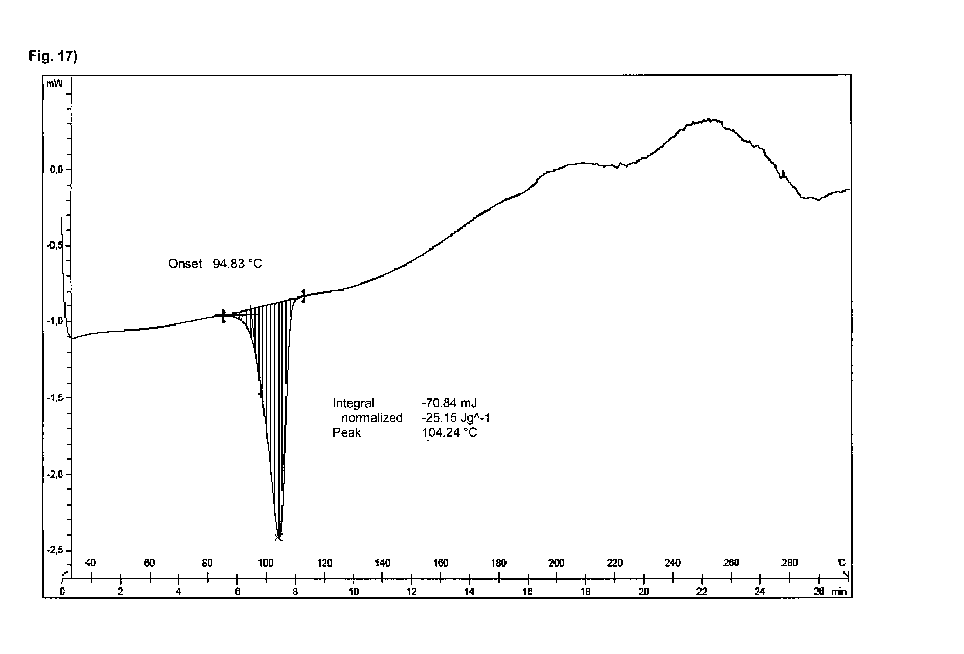

The novel type of crystal of the present invention is characterized for one endothermic peak corresponding to the melting point at an onset 94.83 °C (fusion enthalpy -25.15 J/g) (see figure 17).

### TGA

Thermogravimetric analyses were recorded in a thermogravimetric analyzer Mettler TGA/SDTA851^{e}. A sample of 4.1075 mg was weighed into a 70 µL alumina crucible with a pinhole lid and was heated at 10 °C/min from 30 to 300 °C, under nitrogen (50 mL/min).

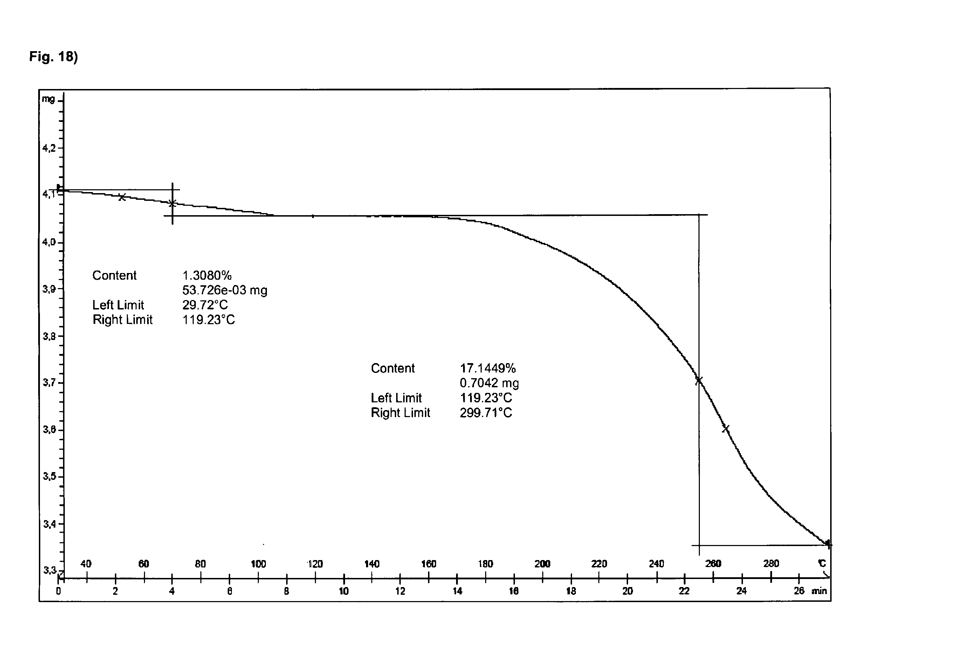

The TG analysis of the crystalline form according to the invention shows a weight loss at temperatures lower than the melting point. It corresponds to water loss (1.3% weight, 0.74 eq. H₂O) (see figure 18). This loss of weight starts at room T° and so the amount of water depends on the RH% of the atmosphere.

### XRPD

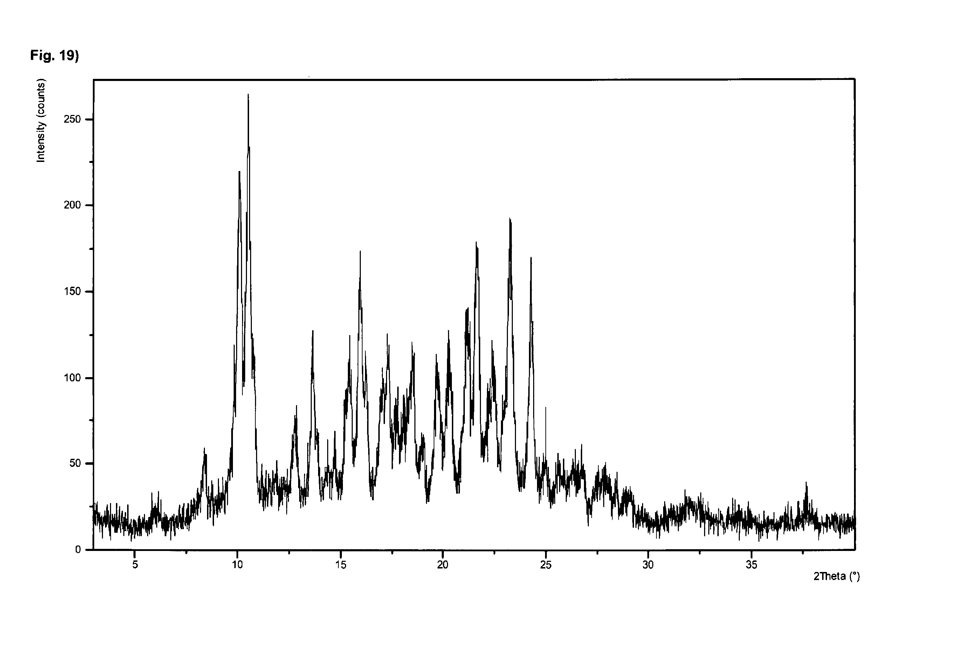

XRPD analysis was performed using a Philips X'Pert diffractometer with Cu K_{α} radiation in Bragg-Brentano geometry. The system is equipped with a monodimensional, real time multiple strip detector. The diffractogram was recorded from 3° to 50° (2θ) at a scan rate of 17.8° per minute (see figure 19).

### List of peaks:

| **2θ (°)** | **d (Å)** | **I (%)** |
|---|---|---|
| 6.07 | 14.56 | 4 |
| 8.42 | 10.50 | 13 |
| 10.11 | 8.75 | 83 |
| 10.53 | 8.40 | 100 |
| 12.82 | 6.90 | 15 |
| 13.62 | 6.50 | 34 |
| 15.41 | 5.75 | 32 |
| 15.97 | 5.55 | 56 |
| 16.25 | 5.46 | 30 |
| 16.99 | 5.22 | 24 |
| 17.34 | 5.12 | 34 |
| 18.54 | 4.79 | 32 |
| 19.02 | 4.67 | 11 |
| 19.68 | 4.51 | 29 |
| 20.26 | 4.38 | 30 |
| 21.11 | 4.21 | 38 |
| 21.69 | 4.10 | 58 |
| 22.47 | 3.96 | 24 |
| 23.31 | 3.82 | 58 |
| 24.28 | 3.67 | 53 |
| 24.97 | 3.57 | 7 |
| 26.82 | 3.32 | 9 |
| 27.78 | 3.21 | 7 |
| 29.05 | 3.07 | 5 |
| 32.13 | 2.79 | 3 |
| 37.73 | 2.38 | 5 |

## Claims

1. A crystalline form comprising a sartan either as a free acid or as a physiologically acceptable salt and at least one beta blocker either in free form or as a physiologically acceptable salt.

2. The crystalline form according to claim 1, wherein the sartan is telmisartan.

3. The crystalline form according to one of claims 1 or 2, wherein the at least one beta blocker is a selective β₁ receptor blocker, preferably
wherein the at least one selective β₁ receptor blocker is selected from acebutolol, atenolol, betaxolol, bisoprolol, celiprolol, esmolol, metoprolol, or nebivolol, preferably is selected from metoprolol, atenolol, or bisoprolol.

4. The crystalline form according to any of claims 1 to 3, wherein the crystalline form comprises in addition to the sartan or telmisartan and the at least one beta blocker an additional compound selected from alcohol, acid or water, preferably selected from ethanol, fumaric acid or water.

5. The crystalline form according to any of claims 1 to 4, wherein the ratio between the sartan or telmisartan and the at least one beta blocker is chosen in such a way that if compared to either telmisartan alone and/or to a mixture of telmisartan and the at least one beta blocker
• the solubility of the crystalline form is increased; and/or
• the dose response of the crystalline form is increased; and/or
• the efficacy of the crystalline form is increased; and/or
• the dissolution of the crystalline form is increased; and/or
• the bioavailability of the crystalline form is increased; and/or
• the stability of the crystalline form is increased; and/or
• the hygroscopicity of the crystalline form is decreased; and/or
• the form diversity of the crystalline form is decreased; and/or
• the morphology of the crystalline form is modulated.

6. The crystalline form according to any of claims 1 to 5, wherein the crystalline form is a salt, preferably wherein the crystalline form is a salt selected from a salt of telmisartan and metoprolol, or a salt of telmisartan and atenolol, more preferably wherein the crystalline form is a salt selected from a salt of telmisartan - metoprolol (1:1), or a salt of telmisartan - atenolol - H₂O (1:1:1).

7. The salt of telmisartan - metoprolol (1:1) according to claim 6, **characterized in that** the endothermic sharp peak corresponding to the melting point has an onset at 131 °C, preferably at 130.9 °C, most preferably at 130.92 °C; and/or
**characterized in that** it shows an X-Ray powder diffraction pattern with peaks [2θ] at 6.84, 13.15, 20.06, 20.36, and 22.71° or at 6.8, 13.2, 20.1, 20.4, and 22.7°, preferably with peaks [2θ] at 6.84, 10.06, 13.15, 16.49, 18.07, 20.06, 20.36, 22.56, and 22.71°; more preferably with peaks [2θ] at 6.04, 6.84, 10.06, 13.15, 13.96, 14.35, 16.15, 16.49, 17.06, 17.76, 18.07, 19.00, 20.06, 20.36, 21.44, 21.65, 22.56, 22.71, 23.53, and 25.12°; most preferably with peaks [2θ] at 6.04, 6.84, 8.46, 10.06, 10.58, 11.74, 12.12, 13.15, 13.71, 13.96, 14.35, 14.60, 15.03, 15.38, 16.15, 16.49, 17.06, 17.76, 18.07, 19.00, 20.06, 20.36, 20.55, 21.25, 21.44, 21.65, 22.56, 22.71, 23.01, 23.53, 24.29, 24.56, 24.91, 25.12, 26.09, 26.63, 27.16, 29.28, 29.89, 30.25, 33.11, 33.72, and 38.19.

8. The salt of telmisartan - atenolol - H₂O (1:1:1) according to claim 6, **characterized in that** the endothermic sharp peak corresponding to the melting point has an onset at 113 °C, preferably at 113.1 °C, most preferably at 113.05 °C; and/or
**characterized in that** it shows an X-Ray powder diffraction pattern with peaks [2θ] at 8.45, 14.67, 17.86, and 19.66 °, or at 8.5, 14.7, 17.9, and 19.7 °, or at 8.45, 14.67, 15.99, 17.86, 19.66, 20.99, and 22.19 °; preferably with peaks [2θ] at 8.45, 14.43, 14.67, 15.99, 17.02, 17.86, 19.14, 19.66, 20.99, 22.19, 25.48, and 25.66 °; more preferably with peaks [2θ] at 8.45, 8.68, 11.95, 12.44, 14.43, 14.67, 15.48, 15.62, 15.99, 17.02, 17.86, 18.07, 19.14, 19.66, 20.56, 20.99, 22.19, 23.48, 25.48, 25.66, and 25.82, °; most preferably with peaks [2θ] at 8.45, 8.68, 9.34, 10.35, 10.60, 11.95, 12.44, 14.43, 14.67, 15.48, 15.62, 15.99, 17.02, 17.47, 17.86, 18.07, 18.81, 19.14, 19.66, 20.56, 20.99, 21.71, 22.19, 22.58, 23.48, 23.80, 24.11, 24.47, 24.90, 25.48, 25.66, 25.82, 26.64, 27.26, 27.89, 28.43, 29.10, 29.76, 30.69, 31.09, 32.16, 34.43, 38.49, and 38.97 °; and/or
**characterized in that** it has a monoclinic unit cell with the following dimensions:
a = 22.76 Å,
b = 9.24 Å, and
c = 22.18 Å,
with an angle β of 113.98 °.

9. The crystalline form according to any of claims 1 to 5, wherein the crystalline form is a co-crystal; preferably wherein the crystalline form is a co-crystal selected from a co-crystal comprising telmisartan and atenolol or a co-crystal comprising telmisartan and bisoprolol, more preferably wherein the crystalline form is a co-crystal selected from a co-crystal of telmisartan - atenolol - EtOH (6:3:2), a co-crystal of telmisartan - bisoprolol - fumaric acid (2: 1: 1), or a co-crystal of telmisartan - bisoprolol - fumaric acid (4:1:2).

10. The co-crystal of telmisartan - atenolol - EtOH (6:3:2) according to claim 9, **characterized in that** the endothermic sharp peak corresponding to the melting point has an onset at 194 °C, preferably at 194.5 °C, most preferably at 194,49 °C; and/or
**characterized in that** it shows an X-Ray powder diffraction pattern with peaks [2θ] at 6.6, 10.7, and 15.7° or at 6.64, 10.67, and 15.66°, or at 6.64, 9.75, 10.67, 15.66, 18.15, 18.49, 20.60, and 20.85 °; preferably with peaks [2θ] at 6.64, 9.75, 10.67, 15.16, 15.66, 17.03, 18.15, 18.49, 19.29, 20.60, and 20.85°; more preferably with peaks [2θ] at 6.09, 6.64, 9.75, 10.67, 13.26, 13.87, 14.68, 15.16, 15.66, 17.03, 18.15, 18.49, 19.29, 20.60, 20.85, 22.64, 23.95, and 25.65°; most preferably with peaks [2θ] at 4.51, 6.09, 6.64, 9.75, 10.67, 11.53, 12.05, 12.66, 13.26, 13.87, 14.68, 15.16, 15.66, 17.03, 17.24, 18.15, 18.49, 19.29, 20.60, 20.85, 21.95, 22.64, 23.35, 23.95, 24.35, 24.77, 25.15, 25.65, 26.12, 26.82, 27.50, 28.60, 31.55, and 33.23°.

11. The co-crystal of telmisartan - bisoprolol - fumaric acid (2:1:1) according to claim 9, **characterized in that** the endothermic peak corresponding to the co-crystal transformation has an onset at 80 °C, preferably at 80.2 °C, most preferably at 80,17 °C; and/or
**characterized in that** the endothermic peak corresponding to the melting point has an onset at 148 °C, preferably at 147.5 °C; and/or
**characterized in that** it shows an X-Ray powder diffraction pattern with peaks [2θ] at 6.2, 15.8, and 22.6 ° or at 6.17, 15.75, and 22.55 °; preferably with peaks [2θ] at 6.17, 14.94, 15.75, and 22.55 °; more preferably with peaks [2θ] at 6.17, 11.21, 14.94, 15.75, 20.10, 21.97, and 22.55 °; most preferably with peaks [2θ] at 6.17, 7.45, 8.93, 11.21, 13.39, 14.94, 15.75, 17.24, 18.56, 19.12, 20.10, 21.97, 22.55, 25.08, and 27.09 °.

12. The co-crystal of telmisartan - bisoprolol - fumaric acid (4:1:2) according to claim 9, **characterized in that** the endothermic peak corresponding to the co-crystal degradation has an onset at 159 °C, preferably at 158.7 °C, most preferably at 158,69 °C; and/or
**characterized in that** the endothermic peak corresponding to the melting point has an onset at 247 °C, preferably at 246.9 °C, most preferably at 246.92 °C; and/or
**characterized in that** it shows an X-Ray powder diffraction pattern with peaks [2θ] at 6.2, 15.8, and 22.0 °, or at 6.18, 15.79, and 21.96 °; preferably with peaks [2θ] at 6.18, 7.47, 15.79, 20.10, 21.96, and 22.51 °; more preferably with peaks [2θ] at 6.18, 7.47, 11.24, 13.39, 14.98, 15.79, 18.59, 19.15, 20.10, 21.96, and 22.51 °; most preferably with peaks [2θ] at 6.18, 7.47, 8.85, 11.24, 12.13, 13.39, 14.98, 15.79, 17.22, 18.59, 19.15, 19.48, 20.10, 20.49, 21.96, 22.51, 24.37, 25.10, 27.27, and 27.62 °.

13. Process for the production of a crystalline form according to any of claims 1 to 12 comprising the steps of:
(a) dissolving or suspending the sartan or telmisartan and at least one beta blocker in a solvent or mixture of solvents,
(b) optionally mixing or stirring the solution or suspension,
(c) optionally before, during or after step (b) keeping the mixed solution/suspension at ambient temperature or above;
(d) optionally filtering-off and/or washing the resulting solid with a solvent, and
(e) drying the solid optionally at ambient temperature and/or vacuum.

14. Medicament comprising at least one crystalline form according to any of claims 1 to 12 and optionally one or more pharmaceutically acceptable excipients.

15. The crystalline form according to any one of claims 1 to 12 for use in the treatment of hypertension, heart failure or myocardial infarction.
